(19) **Europäisches Patentamt European Patent Office Office européen des brevets**

(11) **EP 1 361 264 B1**

(12) **EUROPEAN PATENT SPECIFICATION**

(45) Date of publication and mention of the grant of the patent:
**18.04.2007 Bulletin 2007/16**

(21) Application number: **02700585.9**

(22) Date of filing: **14.02.2002**

(51) Int Cl.:
*A21D 13/08* (2006.01)     *A21D 2/18* (2006.01)
*A21D 2/16* (2006.01)     *A23L 1/03* (2006.01)
*A23L 1/035* (2006.01)     *A23L 1/30* (2006.01)
*A23L 1/48* (2006.01)     *A23L 1/39* (2006.01)
*A23L 1/20* (2006.01)     *A23L 1/052* (2006.01)
*A23D 7/00* (2006.01)     *A61K 31/716* (2006.01)

(86) International application number:
**PCT/JP2002/001264**

(87) International publication number:
**WO 2002/064714 (22.08.2002 Gazette 2002/34)**

(54) **PRODUCTS CONTAINING G(b)-GLUCAN**

PRODUKTE ENTHALTEND G(b)-Glucan

PRODUITS CONTENANT DU G(B)-GLUCANE

(84) Designated Contracting States:
**DE DK FR GB IT NL**

(30) Priority: **15.02.2001 JP 2001037997**
**10.04.2001 JP 2001111089**
**18.04.2001 JP 2001119146**
**18.04.2001 JP 2001119207**
**18.04.2001 JP 2001119345**

(43) Date of publication of application:
**12.11.2003 Bulletin 2003/46**

(73) Proprietor: **Adeka Corporation**
**Tokyo 116-8554 (JP)**

(72) Inventors:
• **TSUBAKI, Kazufumi**
**c/o Asahi Denka Kogyo K. K.**
**Tokyo 116-0012 (JP)**
• **SUGIYAMA, Hiromu**
**c/o Asahi Denka Kogyo K. K.**
**Tokyo 116-0012 (JP)**

• **SHOJI, Yoshikazu**
**c/o Asahi Denka Kogyo K. K.**
**Tokyo 116-0012 (JP)**

(74) Representative: **Forstmeyer, Dietmar et al**
**BOETERS & LIECK**
**Oberanger 32**
**80331 München (DE)**

(56) References cited:
**WO-A-01/30359**          **WO-A1-94/21128**
**WO-A1-99/25198**          **JP-A- 6 065 082**
**JP-A- 10 120 704**          **JP-B2- 6 083 652**
**US-A- 4 231 802**

• **PATENT ABSTRACTS OF JAPAN vol. 1997, no. 05, 30 May 1997 (1997-05-30) -& JP 09 009890 A (TAKEDA CHEM IND LTD), 14 January 1997 (1997-01-14)**
• **DATABASE WPI Section Ch, Week 200333 Derwent Publications Ltd., London, GB; Class D13, AN 2003-345457 XP002314009 -& JP 2002 253143 A (ORGANO CORP) 10 September 2002 (2002-09-10)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

Technical Field:

The present invention relates to a fat and oil composition containing β-glucans extracted from a plant of the Gramineae family. More particularly, it relates to an edible fat and oil composition which is uniformly dispersible in fats and oils and can be used in foods and the like as dispersed in fats and oils to easily supply β-glucans having bioregulatory functions and which is uniformly dispersible throughout a food to improve the taste, texture, etc. of the food and to facilitate development of flavor of the food.

Background Art:

β-Glucans are material attracting attention for applications because of their excellent bioregulatory functions that have recently been analyzed, such as lipid metabolism improving action, intestinal regulatory action, and blood sugar controlling action. Broad application of such material to processed foods will bring extreme benefits, not only contributing to enhancement of functionality of processed foods (addition of value) but matching the expectation of contribution to public health maintenance. β-Glucans are contained in gramineous plants. β-Glucan occurring in gramineous plants, for example, barley and oats are components making cell walls of endosperms in seeds and are distributed almost throughout the seeds. A β-glucan content in barley flour is generally 3 to 10% by weight, while varying according to the parts or species. Structurally, β-glucans are glucose polymers mainly constructed through 1-3-β- and 1-4-β-D-glucopyranose bonds.

JP 09 009 890 discloses the production of a fine particulate gel useful in food by using β-1,3-glucan. US 4,231,802 discloses a method of preparing powders and granules capable of forming a stabilized micro-dispersion of a difficult to disperse material in cold water. This method comprises mixing the difficult to disperse material in liquid form with a matrix comprising by weight from about 70 to about 99 parts of disintegrated β-1,4-glucan. WO 01 30359 discloses a cholesterol lowering and blood lipids lowering composition which can contain β-glucan. WO 99 25198 discloses foodstuffs and drink mixes containing extruded fiber-containing intermediates which can contain β-glucan. WO 94 21128 discloses fat-reduced laminated doughs which can contain β-glucan.

In manufacturing processed foods added with or enriched in β-glucans, for example, foods added with or enriched in barley β-glucans, conceivable methods of fortification include (1) addition of barley grains or flour and (2) addition of β-glucans extracted from barley bran or grains. According to the method (1), it is relatively easy to fortify with β-glucans by adding barley grains or barley flour to a raw material mix which mainly comprises wheat flour and has been used in the manufacture of confectionery products. Nevertheless the method is problematical in that the β-glucan content in the food that can be reached by fortification is limited since the β-glucan content in the added barley grains or flour is about 10% by weight at most and that such addition of barley grains or flour can impair the taste or texture or cause uneven baking thereby to reduce product values. Addition of barley bran that contains β-glucans and other functional components in larger proportions may be conceivable. However, addition of bran is more problematical, easily causing reduction of taste and texture and uneven baking, which results in reduced commercial value. Besides, where added to liquid substances such as fats and oils, barley grains, barley flour and barley bran are difficult to mix and disperse uniformly. It is very difficult to prepare eatable fat and oil compositions containing these materials.

To the contrary, the method (2) which utilizes extracted β-glucans is useful and advantageous in that a β-glucan content in a processed food is arbitrarily adjustable. However, if barley β-glucans, which have high water absorption, is added as such to a raw material mix mainly comprising wheat flour and kneaded with water, they easily form lumps. Such lumps make the mix non-uniform, leading to reduction of quality in taste and texture. Although it is possible to obtain foods having β-glucans dispersed therein relatively uniformly by adding barley β-glucans as previously dissolved in water to a raw material mix (mostly powdered), this method has poor workability and impracticability in the site of manufacture because it is not easy to prepare a uniform aqueous solution. That is, it takes time to dissolve, and the resulting aqueous solution is viscous. In the manufacture of foods added with extract containing β-glucans obtained from gramineous plants such as barley, it has therefore been awaited to establish a process for uniformly dispersing β-glucans in a food to conveniently provide a processed food or to develop such β-glucan materials originated in gramineous plants.

On the other hand, quality modifiers have been in use for a variety of purposes, such as improvement of taste or texture of foods, processing capabilities, cooking capabilities, and workability in food production steps.

For instance, in the production of bakery product dough it is a practice to use an agent for modifying stickiness, softness or hardness of dough to improve workability in dough preparation or shaping. For baking, too, it is required to use modifiers contributory to improvements on product values, such as improvement on volume and springiness and elimination of a bumpy rough surface developed on the baked products. For preservation or storage of bakery products, it is a subject to prevent or retard hardening and deterioration of physical properties, such as texture, with time after baking, and modifiers have been developed to address the subject. For frozen dough, modifiers are indispensable additives for imparting freeze resistance to dough. To meet these purposes, various food additives have been used, including emulsifiers, such as polyglycerol fatty acid esters, fatty acid monoglycerides, and lecithin; oxidizing agents acting to enhance gluten network; enzymes, such as α-amylase and protease serving to modify gluten network; fats and oils containing the components recited above; saccharides, such as sorbitol and trehalose; thickening polysaccharides, such as xanthan gum and pectin; and gelatinized starch (α-starch).

Under these circumstances, today's consumers are highly concerned about safety while pursuing good taste and texture. There is a visible tendency to avoid synthetic additives and seek naturally-occurring substances. It has therefore been keenly desired to establish methods for settling the problems associated with foods by minimizing the amounts of the above-recited additives that may impair taste or texture or hinder manifestation of flavor, particularly synthetic emulsifiers but by using, if possible, only naturally-occurring substances; and to develop additives based on naturally-occurring substances.

Accordingly, an object of the present invention is to provide a β-glucan-containing fat and oil composition which is capable of supplying β-glucans having excellent bioregulatory functionality without impairing taste and texture.

Disclosure of the Invention:

The present invention provides a β-glucan-containing fat and oil composition characterized by containing β-glucans extracted from a gramineous plant.

The present invention provides the β-glucan-containing fat and oil composition which contains a β-glucan having at least two kinds of bonds selected from a 1-2-β-D-glucopyranose bond, a 1-3-β-D-glucopyranose bond, a 1-4-β-D-glucopyranose bond, and a 1-6-β-D-glucopyranose bond.

The present invention provides the β-glucan-containing fat and oil composition which contains a β-glucan having a 1-3-β-D-glucopyranose bond and a 1-4-β-D-glucopyranose bond.

The present invention provides the β-glucan-containing fat and oil composition, wherein the gramineous plant is barley or oats.

The present invention provides a food containing the β-glucan-containing fat and oil composition.

The present invention provides a fat and oil emulsified composition containing the β-glucan-containing fat and oil composition.

The present invention provides a bakery product containing the β-glucan-containing fat and oil composition.

The present invention provides a confectionery product containing the β-glucan-containing fat and oil composition.

The present invention provides a food having a prophylactic action for habitual diseases (or life style-related diseases) containing the β-glucan-containing fat and oil composition.

The present invention provides a drug having a prophylactic action for habitual diseases containing the β-glucan-containing fat and oil composition.

The present invention provides a processed rice, wheat, maize or soybean product containing the β-glucan-containing fat and oil composition.

The present invention provides a liquid food containing the β-glucan-containing fat and oil composition.

The present invention provides a processed food mainly comprising starch and containing the β-glucan-containing fat and oil composition.

Best Mode for Carrying out the Invention:

The present invention will be described hereunder in detail. β-Glucans extracted from gramineous plants (hereinafter also referred to as "extracted β-glucans" or "β-glucan extract") which can be used in the fat and oil composition according to the present invention are not particularly restricted in the manner of extraction. An extracting solvent is added to a gramineous plant as a feed for extraction, and the plant is extracted. Any extract can be used irrespective of the extracting method, form or purity. For example, an extract layer *per se* obtained by solid-liquid separation, a liquid or solid β-glucan concentrate obtained from the extract layer in a conventional manner, and a liquid or solid with increased purity prepared from the extract layer with a conventional purification means are included. It is no problem if the extract contains extracted components other than β-glucans. In the present invention, all these substances are called "β-glucans extracted from a gramineous plant".

Gramineous plants include cereals, such as rices, wheats, maizes, sorghums, barnyard millets, foxtail millets, millets, barleys, oats, and ryes. While a whole plant can be used as a feed for extraction, seeds having relatively high β-glucan contents are preferred. Any of whole grain flour, bran by-produced in grain polishing, malt, germs, and endosperms can be used. Preferred feeds include whole grain flour of barleys or oats, endosperms separated from an outer part of grains of barleys or oats by polishing, brans generated from barleys or oats in polishing, rice bran, and brans and germs separated form wheat or maize by polishing. Still preferred feeds are whole grain flour of barleys or oats, endosperms separated from an outer part of grains of barleys or oats by polishing, and brans generated from barleys or oats in polishing.

It is preferred for the β-glucans of the present invention extracted from gramineous plants to comprise a β-glucan having at least two kinds of bonds selected from a 1-2-β-D-glucopyranose bond, a 1-3-β-D-glucopyranose bond, a 1-4-β-D-glucopyranose bond, and a 1-6-β-D-glucopyranose bond. It is preferred for the β-glucans of the present invention to contain a β-glucan having a 1-3-β-D-glucopyranose bond and a 1-4-β-D-glucopyranose bond.

The method for extracting β-glucans of the present invention from gramineous plants is described below. The β-glucans according to the present invention are water-soluble polymers and capable of being dissolved in water. They are separated by extracting, for example, grain flour of a gramineous plant with 2 to 100 times as much solvent, such as water, warm water, hot water, a salt solution, an aqueous acid or alkali solution or an organic solvent, at an arbitrary temperature for an arbitrary period of time. The extraction system is subjected to solid-liquid separation to obtain β-glucans. β-Glucans obtained by extraction with water, warm water or hot water are preferred. Those extracted with warm water at 4 to 80°C are still preferred. An extraction accelerators and the like may be added in extracting.

More specifically, methods for obtaining high-molecular-weight β-glucans from barley, etc. include a method in which waxy barley is extracted with water (JP-B-4-11197), a method of obtaining β-glucans having a weight average molecular weight of 100,000 to 1,000,000 from barley or oats which comprises alkali extraction, neutralization, and precipitation in an alcohol (JP-B-6-83652), and a method of extracting β-glucans in which bran separated by polishing barley, etc. to a polishing yield of 82% or less is extracted with hot water at 80 to 90°C (JP-A-11-225706). Low-molecular-weight β-glucans obtained by conventional molecular weight reduction of the β-glucans obtained by these methods are also useful. Any known reactions for hydrolyzing polysaccharides is adoptable for molecular weight reduction of β-glucans. For example, it is known that water-soluble polysaccharides are hydrolyzable by heat and pressure application in the presence of an acid. This is made use of for molecular weight reduction of β-glucans. Molecular weight reduction making use of enzymatic hydrolysis is also effective. Useful enzymes include 1,3-β-glucanase. β-Glucans obtained by directly extracting a grain raw material by the method disclosed in WO98/13056, Japanese Patent Application No. 2000-287920, etc. are also usable. The extraction accelerators and the like described in Japanese Patent Application No. 2000-295660 can be used.

The β-glucans extracted from a gramineous plant which can be used in the present invention are high-molecular compounds. While β-glucans having any weight average molecular weight are usable, those having a molecular weight of less than 3,000,000, preferably less than 500,000, still preferably less than 100,000, are suitable because the compatibility with fat and oil increases as the molecular weight decreases. The molecular weight of extracted β-glucans may be reduced in a conventional manner to have improved compatibility with fat and oil, or low-molecular β-glucans may be directly obtained by extraction.

The fats and oils or fat and oil compositions in which the β-glucans extracted from a gramineous plant are dispersed are not particularly limited and include soybean oil, rapeseed oil, mustard oil, cotton seed oil, safflower oil, sesame oil, corn oil, peanut oil, kapok oil, sunflower oil, rice oil, rice bran oil, coconut oil, palm oil, palm kernel oil, linseed oil, castor oil, olive oil, cocoa butter, tung oil, camellia oil, illippe butter, Borneo tallow, Mowrah, sal butter, borage oil, lauric acid fat and oil, hard butter, shea butter, oleic acid-linoleic acid fat and oil, erucic acid oil, linolenic acid oil, conjugate acid oil, oxyacid oil, Cuphea oil, crambe (*Crambe abyssinica*) seed oil, meadowfoam oil, lesquerella (*Lesquerella fenderli*) seed oil, macadamia oil, evening primrose (*Oenothera biennis* and *Oenothera lamarkiana*) seed oil, oil of seeds of borage (*Borago officinalis*) from the Boraginaceae family, oil of seeds of amaranth (*Amaranthus* L.; an annual plant of the Amaranthaceae family), pine seed oil, avocado oil, grapeseed oil, oil of dry microbial cells of the filamentous fungus *Mortierella alpina*, lipid of dry microbial cells of Labyrinthulae belong to Xanthophyta, lipid of dry microbial cells of *Schizochytrium* sp. belonging to Labyrinthulomycota, lipid of *Crypthecodinium cohnii,* butter lipid, ghee butter, milk fat, beef tallow, lard, sheep tallow, fats and oils of other land animals, fish oil, whale oil, codliver oil, fats and oils of other marine animals, short chain fatty acid-containing fats and oils, middle chain fatty acid-containing fats and oils, γ-oryzanol, γ-linolenic acid-containing fats and oils, α-linolenic acid-containing fats and oils, docosahexaenoic acid (DHA)-containing fats and oils, eicosapentaenoic acid (EPA)-containing fats and oils, plant sterol-containing fats and oils, trans acid-containing fats and oils, hydroxy acid-containing fats and oils, conjugated fatty acid-containing fats and oils, polyphenol-containing fats and oils, phospholipid-containing fats and oils, sphingolipids, tocotrienol-containing fats and oils, sitostanol fatty acid-containing fats and oils, n-3 polyunsaturated fatty acid-containing fats and oils, diglycerides, and other vegetable or animal fats and oils; processed oils derived from these fats and oils according to necessity, such as hydrogenated oils, slightly hydrogenated oils, hydrogenation isomerized oils, interesterified oils, and fractionated oils, fats and oils processed by two or more of these processes, and mixtures of two or more of these processed fats and oils. Additionally, disperse systems using these fats and oils as a dispersing medium or a dispersoid, such as emulsions (including W/O emulsions, O/W emulsions, double emulsions, i.e., O/W/O emulsions and W/O/W emulsions, and more complex multiple emulsions) and suspensions are also useful (such disperse systems will hereinafter be included under the category "fats and oils"). Further, foods containing the above-recited fats and oils are also designated "fat and oil compositions" in the present invention.

When added to fats and oils, the form of β-glucans extracted from a gramineous plant is not particularly limited and can be added as such or as dissolved in water or any other water-soluble solvent. In preparing β-glucan-containing emulsions, β-glucans may be dispersed in a previously prepared fat and oil emulsion or be dispersed at the time of emulsification. Addition of β-glucans can be carried out with no difficulty nor fail whether the fat and oil is liquid oil or solid fat.

The fat and oil composition containing β-glucans extracted from a gramineous plant is obtained by adding β-glucans extracted from a gramineous plant to fat and oil or a fat and oil composition, followed by mixing. Mixing means are not particularly limited, and mixing devices, such as a mixer, can be used. In particular, mixing β-glucans extracted from a gramineous plant with fat and oil or a fat and oil composition and keeping the mixture at 50°C or higher for a given time, preferably 5 minutes to 6 hours, still preferably 10 minutes to 2 hours, give a fat and oil composition in which the β-glucans extracted from a gramineous plant is uniformly or thoroughly compatible with the fat and oil. In foods produced by using the β-glucans extracted from a gramineous plant which are in a thoroughly compatible state with fat and oil, the β-glucans of a gramineous plant origin are dispersed more uniformly than in foods produced by directly adding β-glucans extracted from a gramineous plant. As a result, there are produced remarkable effects such that the taste or texture is not impaired, reduction of flavor due to an emulsifier is unexpectedly suppressed, and manifestation of the flavor of a food is improved.

It is a feature of the fat and oil composition of the present invention that β-glucans are very uniformly dispersed in fat and oil by use of various mixing, kneading or stirring machines described later.

Where the fat and oil composition is composed of an oily phase and an aqueous phase as with an emulsion, while it is possible to add β-glucans extracted from a gramineous plant to either the oily phase or the aqueous phase, it is preferred that the extracted components of gramineous plant origin be first dispersed completely in the oily phase and then mixed with the aqueous phase so that the β-glucans extracted from a gramineous plant may exhibit satisfactory compatibility with fat and oil to give a homogeneous fat and oil composition. Thus, a fat and oil composition containing β-glucans extracted from a gramineous plant can be obtained in a short time.

Where β-glucans extracted from a gramineous plant are incorporated into a water-in-oil emulsion, a plasticized water-in-oil emulsion, etc., addition of β-glucans extracted from a gramineous plant may be followed by emulsification, or be simultaneous with emulsification, or be preceded by emulsification as stated above, or be preceded by plasticization. In using solid fat, it may be softened or liquefied by an appropriate method according to necessity, and β-glucans extracted from a gramineous plant is then added thereto. In order to highly uniformly disperse β-glucans extracted from a gramineous plant, it is desirable that 100 parts by weight of powdered β-glucans and 10 to 50 parts by weight of edible fat and oil are mixed, and the mixture is then subjected to rolling or a combination of rolling and conching. Other raw materials, an additional amount of oil, and the like may be added in this state to adjust the β-glucan content in the finally obtained fat and oil composition.

The means for mixing β-glucans extracted from a gramineous plant into fats and oils include various types of machines for mixing, kneading or stirring. Examples are propeller agitators, oscillatory mixers, orifice mixers, paddle agitators, agitation emulsifiers (homomixer), cutter mixers, cokneaders, conches, silent cutters, jet mixers, vacuum agitators, screw mixers, static mixers, cutting mixers, sonicators, kneaders, rolls, Hydrossure, pipeline mixers, universal mixers, pin machines, homogenizers (high-pressure homogenizers), ball cutters, and ribbon mixers. It is preferred to use an agitation emulsifier (homomixer) and/or a homogenizer (high-pressure homogenizer) at a product temperature of 40° to 80°C. After mixing by agitation, the fat and oil composition containing β-glucans extracted from a gramineous plant may be stored as obtained, or emulsified, or rapidly cooled for plasticization. For plasticization, a votator, a combinator, a perfector, a complector, Onreitor etc. can be used. Use of a pin machine at a product temperature of 10°C or lower is preferred. It is also possible that β-glucans extracted from a gramineous plant is added to fat and oil having been emulsified and then processed in a rapid cooling-plasticizing apparatus, such as a votator, a combinator, a perfector, a complector or a scraped-surface heat exchanger, and the mixture is treated by any of the above-described methods to prepare a fat and oil composition containing β-glucans extracted from a gramineous plant.

The content of β-glucans extracted from a gramineous plant in the fat and oil composition of the present invention is desirably 0.01 to 500 parts by weight, preferably 0.1 to 150 parts by weight, still preferably 1 to 100 parts by weight, per 100 parts by weight of the total composition except the β-glucans. Where the content of β-glucans extracted from a gramineous plant is less than 0.01 part by weight, a final product tends to fail to exhibit the functional effects of the β-glucans. If it exceeds 500 parts by weight, the mixture tends to become powdery or lumpy irrespective of the kinds of other ingredients, failing to provide an edible fat and oil composition having β-glucans extracted from a gramineous plant uniformly mixed and dispersed therein. Even after the mixture is processed into a final product, the lumps would remain only to cause non-uniform distribution of the extract.

Where an extract layer obtained by extracting a gramineous plant is used as such without being purified or merely after being powderized or solidified, the β-glucan purity of the extract is preferably as high as possible. An acceptable purity ranges 1 to 100%, and a preferred purity is from 10 to 100%, particularly 20 to 100%.

It is possible to add, to the fat and oil composition containing β-glucans extracted from a gramineous plant of the present invention, food additives or foods such as emulsifiers, gelling agents, thickeners, and stabilizers to prevent the β-glucans from getting distributed non-uniformly due to agglomeration into lumps in the composition. The foods or food additives are not particularly limited as far as they are edible. Examples of the emulsifiers are lecithin, fatty acid monoglycerides, sorbitan fatty acid esters, propylene glycol fatty acid esters, sugar esters, polyoxyethylene sorbitan fatty acid esters, Tween, polyoxyethylene sorbitan trioleate, polyoxyethylene sorbitan monooleate, polyoxyethylene sorbitan monostearate, polyoxyethylene sorbitan monopalmitate, and polyoxyethylene sorbitan monolaurate. Examples of the thickeners and the stabilizers are pullulan, psyllium, gum arabic, gellan gum, glucomannan, guar gum, xanthan gum, tamarind gum, carrageenan, alginic acid salts, farceran, locust bean gum, pectin, curdlan, and low-molecular compounds obtained from these substances, starch, processed starch, gelatinized starch, crystalline cellulose, gelatin, dextrin, agar, and dextran. Additional useful food additives or foods include saccharides, such as glucose, fructose, sucrose, maltose, enzyme-saccharified sugar (thick malt syrup), lactose, reducing starch saccharification products, isomerized liquid sugar, sucrose-coupled malt syrup, oligosaccharides, reducing sugar polydextrose, sorbitol, reduced lactose, trehalose, xylose, xylitol, maltitol, erythritol, mannitol, fructo-oligosaccharides, soybean oligosaccharides, galacto-oligosaccharides, lactosucrose-oligosaccharides, raffinose, lactulose, palatinose-oligosaccharides, stevia, and Aspartame; stabilizers, such as phosphoric acid salts (e.g., hexametaphosphorates, secondary phosphates and primary phosphates) and alkali metal (e.g., potassium or sodium) salts of citric acid; proteins, such as whey proteins, (e.g., α-lactalbumin, β-lactoglobulin, and serum albumin), casein and other milk proteins, low-density lipoprotein, high-density lipoprotein, egg proteins (e.g., phosvitin, livetin, phosphoglycoprotein, ovalbumin, conalbumin, and ovomucoid), wheat proteins (e.g., gliadin, glutenin, prolamine, and glutelin), and other vegetable and animal proteins; inorganic salts, such as sodium chloride, rock salt, sea salt, and potassium chloride; souring agents, such as acetic acid, lactic acid, and gluconic acid; colorants, such as β-carotin, caramel, and Monascus color; antioxidants, such as tocopherol and tea extract; eggs, such as whole eggs, egg yolk, egg white, and enzyme-processed eggs; cereals, such as bread flour, all-purpose flour, and cake flour; beans, such as soybean powder; water, flavors, dairy products, seasonings, pH adjustors, enzymes, food preservatives, shelf life extenders, fruits, fruit juices, coffee, nut pastes, spices, cacao mass, and cocoa powder. Two or more of these additives can be used in combination. The amounts of the additives to be added are not particularly limited. They can be added in general amounts, for example, 0.01 to 15% by weight based on the composition.

The foods to which the fat and oil composition of the present invention is applicable will then be described. The foods that can be used are those containing the aforementioned fat and oil composition containing β-glucans extracted from a gramineous plant as a part or the whole of the fats and oils conventionally employed therefor or those containing β-glucans extracted from a gramineous plant and fats and oils. Included in such foods are not only fat and oil foods exemplified by margarine and shortening but any kinds containing fats and oils, such as bakery products, confectionery products, processed rice products, processed wheat products, processed maize products, processed soybean products, health foods, and medicinal foods. The fat and oil composition containing β-glucans extracted from a gramineous plant according to the present invention can be used as a substitute for a part of, or the whole of, a fat and oil composition used in these foods according to conventional usage whether it is liquid (e.g., salad oil, frying oil, and whipping cream), sol (e.g., liquid shortening), pasty or emulsion (e.g., foamable emulsified fats, dressings, fat spreads, custard cream, and dipping cream) or solid (e.g., shortening, margarine, candies, chocolates, and roux type curry sauce mixes).

Examples of products to which the fat and oil composition is applied include foods, food additives, cosmetics, toiletries, and drugs. The foods include grain-related products, such as those containing wheat flour as a main ingredient and those containing rice as a main ingredient, such as bread, dessert bread, hot dog buns, and Danish pies; pancakes, doughnuts, pizza, *tempura,* and premixes thereof; cookies/biscuits and snacks; noodles, such as raw noodles, dry noodles, packaged instant noodles, instant cup noodles, *udon* noodles (white wheat noodles), buckwheat noodles, Chinese noodles, rice noodles, and pastas; rice products, such as boiled rice, rice cake, sterile boiled rice, retort pouch boiled rice, nonglutinous rice flour, glutinous rice flour, dumplings, rice crackers, and rice chips; and toppings.

The foods also include confectionery products, either Japanese or European, such as chocolates, candies, drops, chewing gums, baked confections, cakes, and sweet bean-jam buns.

The foods also include processed meat products, such as ham, sausage, and hamburgs; and processed marine products, such as steamed fish paste, baked fish paste, fried fish paste, and fish sausage.

The foods also include dairy products, such as butter, cheese, ice cream, and yogurt.

The foods also include beverages, such as alcoholic beverages, e.g., beer, *sake,* whisky, brandy, *sho-chu* (Japanese distilled liquor), distilled liquors, sparkling alcohol, wines, and fruit wines; coffee, tea, green tea, woolong tea, Chinese tea, cocoa, carbonated beverages, nutritious drinks, sports drinks, coffee drinks, lactic acid beverages, fruit juices, and fruit drinks.

The foods also include processed fat and oil products, such as margarine, shortening, mayonnaise, and cream.

The foods also include seasonings and sauces, such as spices, dips, dressings for meat, salad dressings, Worcester sauce, *miso,* soy sauce, sauce mixes, e.g., curry sauce mixes and hashed beef sauce mixes; soups, such as corn soup, potato soup, and pumpkin soup; jams, peanut butter; and toppings.

The foods also include preserved foods, such as canned or bottled foods of fishes and shells, meats, fruits, vegetables, mushrooms, corned beef, jams, tomatoes, etc.; frozen foods; retort pouch foods, such as curry, stew, meat sauce, ma-po tofu, stew of meat with vegetables, soups, and boiled rice; and powdered foods, such as instant powder foods, e.g., powdered beverages, powdered soups, and powdered *miso* soup.

The foods also include special health foods, such as baby foods (e.g., weaning foods), invalid diets (e.g., liquid diets), diets for the old, fat-reducing diets, and supplementary foods.

The foods also include microwave foods ready to be reheated or cooked.

The foods also include staple diets such as bread, noodles, and rice.

The foods also include those easy to take everyday or regularly.

The foods also include those easy to add to other foods.

The foods also include those which tend to be insufficient, those necessary for holding biobalance, and those which, when combined with other foods, result in better nutriment.

The foods also include those, when combined with other foods, result in a synergetic effect.

The fat and oil emulsified composition will be described in more detail.

The fat and oil emulsified composition according to the present invention is not particularly limited as long as it comprises fat and oil, water, and β-glucans extracted from a gramineous plant.

The extracted β-glucan content in the fat and oil emulsified composition is preferably 0.005 to 20% by weight, still preferably 0.5 to 5% by weight.

The fat and oil emulsified composition includes oil-in-water (O/W) emulsions, water-in-oil (W/O) emulsions, and double emulsions, i.e., O/W/O emulsions and W/O/W emulsions, and more complex multiple emulsions. Specific examples of these emulsions are margarine, fat spread, butter cream, custard cream, flour paste, whipped cream, mayonnaise, dressings, white sauce, tartar sauce, milk creams (mainly comprising raw milk, milk or a residue after removing components other than milk fat from milk) used in ice cream, cakes, etc. or with coffee, and cooking creams (raw cream, creme chantily or creme fouette, cream for entremets, cream for patisserie, butter cream, creme patissiere, creme anglaise, and creme Saint Honore). These fat and oil emulsified compositions are obtained with excellent emulsion stability, flavor, and texture. In the case of sour oil-in-water emulsions such as mayonnaise, the effect of softening sourness is manifested to provide a natural vinegar flavor.

The amount of fat and oil is not particularly limited and decided according to the type of the emulsified compositions. For example, the fat and oil content of oil-in-water emulsified compositions is preferably 4 to 85% by weight, still preferably 4 to 70% by weight, particularly preferably 4 to 50% by weight, and that of water-in-oil emulsified compositions is preferably 40 to 95% by weight, still preferably 45 to 90% by weight, particularly preferably 50 to 80% by weight.

The water content is not particularly limited and decided according to the type of the emulsified compositions. For example, the water content in oil-in-water emulsified compositions is preferably 15 to 95% by weight, still preferably 30 to 95% by weight, and that in water-in-oil emulsified compositions is preferably 5 to 45% by weight, still preferably 10 to 30% by weight.

The fat and oil emulsified compositions containing β-glucans extracted from a gramineous plant are prepared by adding β-glucans to the aqueous phase and/or the oily phase, and the aqueous phase and the oily phase are emulsified in a usual manner.

If desired, the resulting fat and oil emulsified composition of the present invention may be homogenized by means of a homogenizing machine, such as a valve homogenizer, a homomixer, and a colloid mill. If desired, the fat and oil emulsified composition may be subjected to pasteurization or heat sterilization treatments, such as UHT treatments by a direct heating system (e.g., injection or infusion) or an indirect heating system by use of a plate, tubular or scraped-surface heat exchanger, HTST treatments, a batch treatment, a retort treatment, and microwave heating, or cooking on the fire. According to necessity, the composition may be subjected to re-homogenization. If necessary, the composition may be subjected to a cooling operation such as rapid cooling or slow cooling. If necessary, the composition may be subjected to aging. Further, the fat and oil emulsified composition thus obtained may be refrigerated or frozen for storage, if needed.

In preparing the fat and oil emulsified composition of the present invention, emulsification, homogenization, mixing or preparation of raw materials can be carried out with mixing, kneading or stirring equipment of various kinds commonly employed with no particular restriction. Examples are propeller agitators, oscillatory mixers, orifice mixers, paddle agitators, agitation emulsifiers (homomixer), cutter mixers, cokneaders, conches, silent cutters, jet mixers, vacuum agitators, screw mixers, static mixers, cutting mixers, sonicators, kneaders, rolls, Hydrossure, pipeline mixers, universal mixers, pin machines, colloid mills, homogenizers (high-pressure homogenizers), jet homogenizers, ball cutters, and ribbon mixers. It is preferred to use an agitation emulsifier (homomixer) and/or a homogenizer (high-pressure homogenizer), a jet homogenizer or a colloid mill. Equipment used for heating or cooling includes indirect heating systems using a scraped surface heat exchanger, e.g., Contherm scraped surface heat exchanger (manufactured by Tetra Laval Food), Thermoclinder (manufactured by Iwai Kikai), a surface scraped heat exchanger (manufactured by Izumi Food Machinery Co., Ltd.), CP-Rotorpro scraped surface heat exchanger (manufactured by APV) or Terlotherm scraped surface heat exchanger (manufactured by Terlet), and steam injection direct heating systems, e.g., Steam Nozzle (manufactured by Iwai Kikai), Noritake Cooker (manufactured by Noritake Company), and Steam Injection (manufactured by Tetra Pack).

The fat and oil emulsified composition of the present invention exhibits excellent emulsion stability without using conventional emulsifiers, particularly synthetic emulsifiers, and resistance to freezing or microwave cooking and has good taste and texture and, in addition, a bioregulatory function. As a matter of course, the composition may contain other components effective on emulsification and emulsion stabilization, such as natural emulsifiers, enzymatically treated substances, and thickening polysaccharides, e.g., substances originated in eggs, milk, soybeans or gramineous cereals or enzyme-treated substances thereof.

Examples of such natural emulsifiers or enzymatically treated substances which can be used as an emulsifier include lecithins (e.g., soybean lecithin and egg yolk lecithin), eggs (whole eggs, egg white or egg yolk), milk, ground or dried soybeans, flours of grains, such as wheat and barley, proteins separated from these food materials such as egg protein, defatted milk powder, milk protein, whey proteins, (e.g., $\alpha$-lactalbumin, $\beta$-lactoglobulin, and serum albumin), milk serum protein, casein, sodium casein, and other milk proteins, egg white albumin, ovomucoid, low-density lipoprotein, high-density lipoprotein, egg proteins (e.g., phosvitin, livetin, phosphoglycoprotein, conalbumin, and ovomucoid), proteins of grain origin, such as wheat proteins (e.g., gluten and gliadin), zein, glutenin, and prolamine, soybean protein isolate, and other vegetable and animal proteins, protein-polysaccharide complexes, and substances left after removing components other than milk fat from raw milk, processed milk, concentrated milk, raw sheep milk, etc. Enzyme-treated substances obtained by contact reaction between these materials with enzymes, for example, soybean protein hydrolyzate, wheat protein hydrolyzate, milk protein hydrolyzate, and egg protein hydrolyzate, are also useful. One or more of these substances can be used in combination. The amount of these substances to be added is not particularly limited and can be varied appropriately. They are added in generally adopted amounts, for example, 0.01 to 15% by weight based on the composition of the present invention.

Any enzyme that is expected to modify the materials such as eggs, milk, soybeans and grains to improve emulsifying capabilities can be used to obtain the above-mentioned enzyme-treated substances. Such enzymes include amylases, e.g., $\alpha$-amylase, $\beta$-amylase, and glucoamylase), cellulases (e.g., cellulase and hemicellulase), proteases, lipases (e.g., lipase and phospholipase), glucose oxidase, lipoxygenase, lactase, invertase, pentonase, pectinase, catalase, ascorbic acid oxidase, sulfhydryl oxidase, and hexose oxidase. The term "amylase" denotes one or more amylases selected from the group consisting of $\alpha$-amylase, isoamylase, and glucoamylase. Amylases produced by microorganisms belonging to the genera Bacillus, Pseudomonas, Aspergillus, Rhizopus and Klebsiella are preferred.

Flour of grains includes crude or refined flour of rices, wheats, maizes, sorghums, barnyard millets, foxtail millets, millets, barleys, oats, and ryes. Wheat flour includes bread flour, less strong bread flour, allpurpose flour, cake flour, and duram flour. Buckwheat flour, soybean flour are also usable.

The thickening polysaccharides include pullulan, psyllium, gum arabic, gellan gum, glucomannan, guar gum, xanthan gum, tamarind gum, carrageenan, alginic acid alkali metal salts, farceran, locust bean gum, pectin, curdlan, crystalline cellulose, CMC, gelatin, dextrin, agar, dextran, and low-molecular compounds obtained therefrom. These polysaccharides may be used either individually or as a combination of two or more thereof. A preferred content of the thickening polysaccharides is 0.1 to 5% by weight, particularly 0.5 to 3% by weight. With a thickening polysaccharide content less than 0.1% by weight, the composition tends to have poor shape retention. With contents exceeding 5% by weight, the composition tends to be hard and hardly melt in the mouth.

It is possible to add, to the fat and oil emulsified composition of the present invention, food additives or foods such as emulsifiers, gelling agents, thickeners, and stabilizers other than those described above as long as the effects of the present invention are not impaired. The foods or food additives are not particularly limited as far as they are edible. Examples of the emulsifiers are fatty acid monoglycerides, sorbitan fatty acid esters, propylene glycol fatty acid esters, sugar esters, polyoxyethylene sorbitan fatty acid esters, Tween, polyoxyethylene sorbitan trioleate, polyoxyethylene sorbitan monooleate, polyoxyethylene sorbitan monostearate, polyoxyethylene sorbitan monopalminate, and polyoxyethylene sorbitan monolaurate. Examples of the thickeners and stabilizers are starch, modified starch, and gelatinized starch. Additional useful food additives or foods include saccharides, such as glucose, fructose, sucrose, maltose, enzyme-saccharified sugar (thick malt syrup), lactose, reducing starch saccharification products, isomerized liquid sugar, sucrose-coupled malt syrup, oligosaccharides, reducing sugar polydextrose, sorbitol, reduced lactose, trehalose, xylose, xylitol, maltitol, erythritol, mannitol, fructo-oligosaccharides, soybean oligosaccharides, galacto-oligosaccharides, lactosucrose, raffinose, lactulose, palatinose-oligosaccharides, stevia, and Aspartame; stabilizers, such as phosphoric acid salts (e.g., hexametaphosphoates, secondary phosphates and primary phosphates) and alkali metal (e.g., potassium or sodium) salts of citric acid; inorganic salts, such as sodium chloride, rock salt, sea salt, and potassium chloride; souring agents, such as acetic acid, lactic acid, gluconic acid, citric acid, succinic acid, malic acid, and ascorbic acid; colorants, such as $\beta$-carotin, caramel, and Monascus color; antioxidants, such as tocopherol and tea extract; flavors, such as milk flavor, vanilla flavor, vanilla essence, chocolate flavor, strawberry flavor, and cheese flavor; dairy products, seasonings, pH adjustors, enzymes, food preservatives, shelf life extenders, fruits, fruit juices, coffee, nut pastes, spices, cacao mass, and cocoa powder. Two or more of these additives can be used in combination. The amounts of the additives to be added are not particularly limited. They can be added in general amounts, for example, 0.01 to 15% by weight based on the composition of the present invention.

It is particularly preferred to add tocopherol to improve antioxidation stability of the fat and oil emulsified composition.

Application of the fat and oil compositions to bakery products will then be described. The bakery products contain the aforementioned fat and oil composition containing β-glucans extracted from a gramineous plant. The bakery products include those produced by (a) preparing dough by mixing the fat and oil composition as a part or the whole of conventional fat and oil with water and, in addition, secondary raw materials suited to the product, such as fats and oils, emulsions, sugars, dairy products, eggs, yeast, yeast food, oxidizing agents, reducing agents, various enzymes, and emulsifiers, which are added either all at once or in divided portions and (b) subjecting the resulting dough, either as prepared or after fermentation, to a heating treatment, such as steaming, baking, frying, and boiling, or (b') preserving the resulting dough by refrigeration or freezing and then (b) subjecting the dough to a heating treatment, such as steaming, baking, frying, and boiling. The bakery products include breads (e.g., pancakes, crepe, sponge cakes, doughnuts, dessert bread, loaves of bread, hot dog buns, Danish pies, French bread, rye bread, hard rolls, and Danish pastries), pies, *kasutera* (Japanese style sponge cake), butter cakes, puff pastries, waffles, biscuits/cookies, crackers, and fermented confections. The method of preparing dough is not particularly limited, except that a part of or the whole of the fat and oil which has been conventionally used in dough preparation is substituted with the fat and oil composition containing β-glucans extracted from a gramineous plant according to the present invention. In making bread as an example of the bakery products, bread dough is prepared from general raw materials of bread, such as wheat flour, water, yeast, sugar, and edible salt, and the edible fat and oil composition containing β-glucans extracted from a gramineous plant by a known dough preparation method. For instance, after mixing the other materials, the fat and oil composition containing β-glucans extracted from a gramineous plant may be folded into the mix. The dough is then fermented, shaped, and baked in a usual manner. Similarly, the fat and oil composition containing β-glucans extracted from a gramineous plant can be used as a substitute for a part of or the whole of fat and oil for folding (roll-in fat and oil) or fat and oil for dough and batter in making folded pies; or pieces of fat and oil in the form of chips, straws, etc. in making pie crusts; or foamable emulsified fat and oil or liquid oil for cakes in making sponge cakes.

Where the production of bakery products involves a baking step, if barley grains or barley flour is added as such as a β-glucan supply source or even when β-glucans extracted from a gramineous plant is added to dough or a powder mix, which is then kneaded into dough, lumps are easily formed in the dough. Dough having such lumps only provides a bakery product with a rough or grainy texture or a strange texture due to non-uniformity of the water content or hardness. To the contrary, use of the fat and oil composition containing β-glucans extracted from a gramineous plant provides dough having the β-glucans extracted from a gramineous plant uniformly dispersed therein with very few lumps, which finally gives a baked product with a good texture, not only free from a strange texture but with greatly improved softness. The fat and oil composition of the present invention is preferably used in an amount of 0.05 to 30% by weight, in terms of the β-glucans extracted from gramineous plants, based on the grain flour content of the bakery product.

In using the fat and oil composition in the bakery products, the bakery products can additionally contain at least one additive selected from the group consisting of an emulsifier, an oxidizing agent, and an enzyme and a glucide having at least one of an 1-2-α-D-glucopyranose bond, an 1-3-α-D-glucopyranose bond, an 1-4-α-D-glucopyranose bond, and an 1-6-α-D-glucopyranose bond, D-fructose or a saccharide containing D-fructose.

The emulsifier is preferably at least one selected from fatty acid monoglycerides, diacetyltartaric esters, and sucrose fatty acid esters. Still preferred are diacetyltartaric esters, such as diacetyltartaric acid monoglyceride and diacetyltartaric acid diglyceride. Lecithin, sorbitan fatty acid esters, propylene glycol fatty acid esters, sugar esters, and the like are also useful as the emulsifier.

The emulsifier is preferably used in an amount of 1 to 300 parts by weight per 100 parts by weight of the β-glucans extracted from a gramineous plant and 0.01 to 5% by weight, particularly 0.08 to 1% by weight, based on the grain flour in the bakery product.

The diacetyltartaric acid monoglyceride, which is preferably used as the emulsifier, is not particularly limited by the process of preparation and is usually obtained by reacting diacetyltartaric anhydride with a mixture of a monoglyceride and a diglyceride in the presence of acetic acid or esterifying a mixture of a monoglyceride and a diglyceride with tartaric acid and acetic acid in the presence of acetic anhydride. Accordingly, the diacetyltartaric acid monoglyceride which is preferably used in the present invention is usually obtained as containing diacetyltartaric acid diglyceride. Diacetyltartaric acid monoglyceride in an aqueous solution or an oil-in-water emulsion has a pH ranging from about 1.3 to 1.9. The composition used in the bakery products of the present invention preferably has its pH adjusted within 3.0 to 10.0, particularly 4.5 to 8.5, to improve stability. If necessary, the pH is adjusted by addition of a base or a salt having a pH adjusting action. Any base or salt that has a pH adjusting action and is permitted to be added to foods can be used. Preferred bases or salts include such bases as ammonia, calcium hydroxide, sodium hydroxide, and potassium hydroxides; sodium, potassium, ammonium or calcium salts of acetic acid, carbonic acid, phosphoric acid, succinic acid, glutamic acid, ascorbic acid, tartaric acid, etc.; and sodium hydrogencarbonate.

The oxidizing agent which can preferably be used in the bakery products is at least one selected from cystine and ascorbic acid. Ascorbic acid is still preferred.

The oxidizing agent is preferably used in an amount of 0.01 to 15 parts by weight per 100 parts by weight of the β-glucans extracted from a gramineous plant and 0.0001 to 0.2% by weight, particularly 0.0005 to 0.1% by weight, based on the grain flour in the bakery product.

The enzymes which can be used in the bakery products include any kind having improving effects in breadmaking. At least one enzyme selected from the group consisting of amylases, proteases, cellulases, and hemicellulases is preferably used. Other useful enzymes include lipases, glucose oxidase, lipoxigenase, lactase, invertase, pentonase, pectinase, catalase, ascorbic acid oxidase, sulfhydryl oxidase, hexose oxidase, and transglutaminase.

The enzyme content varies depending on the purity of enzyme preparations or the kind of the enzyme. In using a commercially available enzyme preparation, for example, an advisable amount is 0.01 to 100 parts by weight, preferably 0.05 to 30 parts by weight, per 100 parts by weight of the β glucans extracted from a gramineous plant.

The enzymes to be used will be described in greater detail. The term "amylase" denotes one or more amylases selected from the group consisting of α-amylase, isoamylase, and glucoamylase. Amylases produced by microorganisms belonging to the genera Bacillus, Pseudomonas, Aspergillus, Rhizopus and Klebsiells are preferred. Commercially available amylase preparations can be utilized. Commercially available α-amylase preparations include Amylase AD Amano and AH Amano from Amano Seiyaku K.K.; Termamyl and BAN from Novo Nordisk Bioindustry Ltd.; Uniase BM-8 from Yakult Honsha Co., Ltd.; Spitase HS, HK, PN-4, AI, and LH from Nagase Seikagaku Kogyo K.K.; Sumizyme L from Shin-Nihon Kagaku Kogyo K.K.; Kleistase from Daiwa Fine Chemicals Co., Ltd.; Rohalase AT from Röhm Enzyme GmbH; liquefied enzymes T, K, and SS from Ueda Kagaku K.K.; Biozyme C and L from Amano Seiyaku K.K., and Uniase L from Yakult Honsha Co., Ltd. Commercially available isoamylase preparations include Plullanase Amano and DB-1 from Amano Seiyaku K.K.; and Promozyme from Novo Nordisk Bioindustry Ltd. Commercially available glucoamylase preparations include Glucozyme NL and AF6 from Amano seiyaku K.K.; Sumizyme S, SG, AN, AD, and AL from Shin-Nihon Kagaku Kogyo K.K.; AMG and Dextrozyme from Novo Nordisk Industry Ltd.; Glucozyme from Nagase Seikagaku Kogyo K.K.; Uniase K from Yakult Pharmaceutical Ind. Co., Ltd.; Glutase 600 and AD from Hankyu Bioindustry K.K.; Kokugen and Daizyme from Daiwa Fine Chemicals Co., Ltd.; and Kokulase G2 and Sanzyme from Sankyo Co., Ltd. All these enzyme names are trade names.

The proteases can be of any origin, such as the genus Bacillus, Thermus or Aspergillus, plants, and animals. Preferred proteases are of the genus Bacillus, Thermus or Aspergillus or plants. The proteases can be used either individually or as a combination of two or more thereof. Commercially available protease preparations can be utilized. Examples of commercially available protease preparations are Protease N Amano, P Amano, and S Amano from Amano Seiyaku K.K.; Alkalase and Neutrase from Novo Nordisk Bioindustry Ltd.; Sumizyme LP from Shin-Nihon Kagaku Kogyo K.K.; and Thermoase from Daiwa Fine Chemicals Co., Ltd. All the enzyme names recited above are trade names. Pepsin, trypsin, chymotrypsin, papain, and chymosin (rennet) are also usable.

The cellulases preferably include those originated in the genus Trichoderma, Aspergillus or Fusarium. Commercially available various cellulase preparations can be made use of either individually or as a combination of two or more thereof. Examples of such preparations are Cellulase A and T from Amano Seiyaku K.K.; Sumizyme AC and C from Shin-Nihon Kagaku Kogyo K.K.; Cellulase XP and Celluzyme from Nagase Seikagaku Kogyo K.K.; Cellulase Onozuka and Y-NC from Yakult Pharmaceutical Ind. Co., Ltd.; Meicelase TP, TL and HP from Meiji Seika Kaisha, Ltd.; Driserase from Kyowa Hakko Kogyo Co., Ltd.; and GODO-TCL and TCL-H from Godo Shusei Co., Ltd. All the enzyme names recited above are trade names.

Commercially available various hemicellulase preparations can be used as the hemicellulase either individually or as a combination of two or more thereof. Examples of the hemicellulase preparations are Hemicellulase Amano from Amano Seiyaku K.K.; Sumizyme X from Shin-Nihon Kagaku Kogyo K.K.; Olivex from Novo Nordisk Bioindustry Ltd.; Cellulosin HC, B, TP25 and GM5 from Hankyu Bioindustry K.K.; and GODO-TXL from Godo Shusei Co., Ltd. All these enzyme names are trade names.

The lipases preferably include those of the genus Aspergillus, Rhizopus or Pseudomonas origin. Various commercially available lipase preparations can be used either individually or as a combination of two or more thereof. Such lipase preparations include Lipase A Amano or P Amano from Amano Seiyaku K.K.; and Lipase (Saiken) from Nagase Seikagaku Kogyo K.K. All these enzyme names are trade names.

The glucide having at least one of an 1-2-α-D-glucopyranose bond, an 1-3-α-D-glucopyranose bond, an 1-4-α-D-glucopyranose bond, and an 1-6-α-D-glucopyranose bond which can be used in the bakery products includes starch and glycogen, which are 1-4-α-glucans having an 1-4-α-D-glucopyranose bond; dextran, which is an 1-6-α-glucan having an 1-6-α-D-glucopyranose bond, and mucin which is an 1-3,1-6-α-glucan having an 1-3-α-D-glucopyranose bond and an 1-6-α-D-glucopyranose bond.

A preferred glucide content is such that the ratio of the β-glucans extracted from a gramineous plant to the glucide ranges from 1/1000 to 0.35/1 by weight.

The D-fructose or a saccharide containing D-fructose which can be used in the bakery products includes purified D-fructose, honey, sugar, isomerized sugar, and juices of pears, watermelons, etc.

A preferred content of D-fructose or the saccharide containing D-fructose is such that the ratio of the β-glucans extracted from a gramineous plant to the D-fructose or the saccharide containing D-fructose ranges from 1/40 to 15/1 by weight. With this amount, the dough is easy to work with, and bakery products with a good texture, softness, and good volume can be obtained. Even when frozen, the dough provides bakery products excellent in anti-staling properties. Where D-fructose or the saccharide containing D-fructose is used in an amount of 0.1 part by weight or more in terms of D-fructose per 100 parts by weight of the grain flour of the bakery product, the dough is easy to work with, and bakery products with a good texture, softness, and good volume can be obtained. Even when frozen, the dough provides bakery products excellent in anti-staling properties.

For use in bakery products, the fat and oil composition can contain various additives and vehicles.

Such additives and vehicles include food additives and foods, such as grain flour, yeast, yeast food, fat and oil, a fat and oil emulsified composition (e.g., a water-in-oil emulsion or an oil-in-water emulsion), wheat gluten, expanding agents (e.g., ammonium hydroge4ncarbonate and sodium hydrogencarbonate), bleaching agents (e.g., ammonium persulfate), water, an aqueous solution containing an alcohol, a polyhydric alcohol, a salt, an acid, an alkali, etc., gelling agents, thickeners, and stabilizers. These additives are not particularly limited as far as they are edible. For examples, fats and oils which can be added include soybean oil, rapeseed oil, mustard oil, cotton seed oil, safflower oil, sesame oil, corn oil, peanut oil, kapok oil, sunflower oil, rice oil, rice bran oil, coconut oil, palm oil, palm kernel oil, linseed oil, castor oil, olive oil, cocoa butter, tung oil, camellia oil, illippe butter, Borneo tallow, Mowrah, sal butter, borage oil, lauric acid fat and oil, hard butter, shea butter, oleic acid-linoleic acid fat and oil, erucic acid oil, linolenic acid oil, conjugate acid oil, oxyacid oil, Cuphea oil, crambe (*Crambe abyssinica*) seed oil, meadowfoam oil, lesquerella (*Lesquerella fenderli*) seed oil, macadamia oil, evening primrose (*Oenothera biennis* and *Oenothera lamarkiana*) seed oil, oil of seeds of borage (*Borago officinalis*) from the Boraginaceae family, oil of seeds of amaranth (*Amaranthus* L.; an annual plant of the Amaranthaceae family), pine seed oil, avocado oil, grapeseed oil, oil of dry microbial cells of the filamentous fungus *Mortierella alpina,* lipid of dry microbial cells of Labyrinthulae belong to Xanthophyta, lipid of dry microbial cells of *Schizochytrium* sp. belonging to Labyrinthulomycota, lipid of *Crypthecodinium cohnii,* butter lipid, ghee butter, milk fat, beef tallow, lard, sheep tallow, fats and oils of other land animals, fish oil, whale oil, codliver oil, fats and oils of other marine animals, short chain fatty acid-containing fats and oils, middle chain fatty acid-containing fats and oils, γ-oryzanol, γ-linolenic acid-containing fats and oils, α-linolenic acid-containing fats and oils, docosahexaenoic acid (DHA)-containing fats and oils, eicosapentaenoic acid (EPA)-containing fats and oils, plant sterol-containing fats and oils, trans acid-containing fats and oils, hydroxy acid-containing fats and oils, conjugated fatty acid-containing fats and oils, polyphenol-containing fats and oils, phospholipid-containing fats and oils, sphingolipids, tocotrienol-containing fats and oils, sitostanol fatty acid-containing fats and oils, n-3 polyunsaturated fatty acid-containing fats and oils, diglycerides, and other vegetable or animal fats and oils; processed oils derived from these fats and oils according to necessity, such as hydrogenated oils, slightly hydrogenated oils, hydrogenation isomerized oils, interesterified oils, and fractionated oils, fats and oils processed by two or more of these processes, and mixtures of two or more of these processed fats and oils. Additionally, emulsions (including W/O emulsions, O/W emulsions, double emulsions, i.e., O/W/O emulsions and W/O/W emulsions, and more complex multiple emulsions) of these edible fats and oils are also useful. Examples of the thickeners and stabilizers are pullulan, psyllium, gum arabic, gellan gum, glucomannan, guar gum, xanthan gum, tamarind gum, carrageenan, alginic acid salts, farceran, locust bean gum, pectin, curdlan, and low-molecular compounds obtained from these substances, starch, modified starch, gelatinized starch, crystalline cellulose, gelatin, dextrin, and agar. Additional useful food additives or foods include saccharides, such as glucose, maltose, enzyme-saccharified sugar (thick malt syrup), lactose, reducing starch saccharification products, isomerized liquid sugar, sucrose-coupled malt syrup, oligosaccharides, reducing sugar polydextrose, sorbitol, reduced lactose, trehalose, xylose, xylitol, maltitol, erythritol, mannitol, fructo-oligosaccharides, soybean oligosaccharides, galacto-oligosaccharides, lactosucrose-oligosaccharides, raffinose, lactulose, palatinose-oligosaccharides, stevia, and Aspartame; stabilizers, such as phosphoric acid salts (e.g., hexametaphosphorates, secondary phosphates and primary phosphates) and alkali metal (e.g., potassium or sodium) salts of citric acid; proteins, such as whey proteins, (e.g., α-lactalbumin, β-lactoglobulin, and serum albumin), casein and other milk proteins, low-density lipoprotein, high-density lipoprotein, egg proteins (e.g., phosvitin, livetin, phosphoglycoprotein, ovalbumin, conalbumin, and ovomucoid), wheat proteins (e.g., gliadin, glutenin, prolamine, and glutelin), and other vegetable and animal proteins; inorganic salts, such as sodium chloride, rock salt, sea salt, and potassium chloride; souring agents, such as acetic acid, lactic acid, and gluconic acid; colorants, such as β-carotin, caramel, and Monascus color; antioxidants, such as tocopherol and tea extract; eggs, such as whole eggs, egg yolk, egg white, and enzyme-processed eggs; cereals, such as bread flour, allpurpose flour, and cake flour; beans, such as soybean powder; water, flavors, dairy products, seasonings, pH adjustors, food preservatives, shelf life extenders, fruits, fruit juices, coffee, nut pastes, spices, cacao mass, and cocoa powder. Two or more of these additives can be used in combination or in the form of a complex thereof.

In addition to the above-mentioned materials, any other material that is usually used in bakery products and bakery product dough can be used in the bakery products and bakery product dough according to the present invention. The present invention is applicable to any breadmaking processes, such as a sponge-dough process and a straight process. The time of adding the fat and oil composition of the present invention is not limited. Where the sponge-dough process is applied, the fat and oil composition may be added in the stage of preparing a sponge or in the stage of preparing a dough.

In the present invention it is preferred that the starch in the bakery product dough be partially gelatinized into α-starch before baking to enhance a favorable flavor or texture. The partial gelatinization can be effected by adding previously gelatinized starch of wheat, rice, maize, potato, cassava, taro, etc. or a raw material containing it to the dough or by adding starch or a raw material containing starch to the dough and gelatinizing the starch by heating, addition of hot water, steaming or roasting. For example, hot water can be added to part of wheat flour to convert it into α-starch, or part of wheat flour may be steamed.

Where applied to non-yeast bakery products, such as biscuits and pizza crusts, the fat and oil composition of the present invention gives an increased crispy texture.

The fat and oil composition applied to bakery products supplies β-glucans having excellent bioregulatory functions without being accompanied with reductions of taste and texture. Further, since the fat and oil composition produces the same effects as have been obtained by the conventional additives having various quality improving effects, it makes it possible to reduce the amounts of the conventional additives to be used, making contribution to improvement in taste and texture of bakery products without impairing the flavor of the products. Furthermore, the fat and oil composition makes it feasible to provide bakery products which meet the consumers's trends to naturally occurring substances, safety, and health.

Application of the fat and oil composition to confectionery products will then be described. In an embodiment of confectionery product production, dough is prepared by using the fat and oil composition containing β-glucans extracted from gramineous plants of the present invention as a part of or the whole of conventionally employed fats and oils, and the resulting dough is further processed. Confectionery products according to this embodiment include deep-fried products such as snacks and doughnuts and steamed products, such as steamed cakes and bean-jam buns. Confectionery products in another embodiment include candies, gums, chocolates, and tablets prepared by mixing the oil and fat composition containing β-glucans extracted from a gramineous plant with sugar, flavors, and the like and, if necessary, solidifying and molding the mixture. Cold desserts, such as ice cream and sherbet, are also included under confectionery products.

In making confectionery products, where weight is put on not only flavor but taste, particularly sweetness, it is important to eliminate lumps. Even a very small lump would cause a strange feeling and ruin the commercial value. Since the fat and oil composition according to the present invention previously contains the β-glucans extracted from a gramineous plant uniformly, even when it is added to and mixed with a premixed material, there is provided a final confectionery product which contains the β-glucans extracted from a gramineous plant in a uniformly dispersed state with no lumps, gives no strange feeling, and has a good flavor.

The fat and oil composition according to the present invention can be added to foods or drugs containing a food ingredient having a prophylactic action for habitual diseases (or life style-related diseases) to enhance the action. Example of such foods or drugs are those containing unsaturated higher fatty acids regulating a blood lipid concentration (e.g., EPA and DHA), plant sterols regulating blood serum cholesterol and esters thereof, diacylglycerol, γ-linolenic acid, α-linolenic acid, beet fiber, corn (maize) fiber, psyllium seed coat, tea polyphenol, lecithin; dried bonito peptide, sardine peptide, casein dodecapeptide, and soybean protein isolate which are effective in lowering blood pressure; and lactic acid bacteria, gluconic acid, oligosaccharides, and various dietary fibers which improve the intestinal environment to regulate the intestines. Further, foods or drugs with enhanced bioregulatory functions can be obtained by adding to the fat and oil composition of the present invention substances known to have health improving functionality, such as Chlorella, spirulina, propolis, chitin, chitosan, nucleic acids, leyss (*Ganoderma lucidum*), agaricus, ginkgo leaf extract, lakanka (Lo Hon Go), turmeric, garcinia, apple fiber, gymnema, collagen, blueberry, aloe, saw palmetto, plant fermentation enzyme, soybean isoflavon, chlorophyll, royal jelly, Asian ginseng, prune, and herbs, such as chamomile, thyme, sage, peppermint, lemon balm, mallow, oregano, cat nip tea, yarrow, and hibiscus.

When added to processed foods of rice, wheat, maize or soybeans, the fat and oil composition of the present invention is capable of imparting or enhancing functionality of these foodstuffs. Examples of such processed foods are rice products (e.g., boiled rice, rice cake, retort pouch boiled rice, frozen boiled rice, and sterile boiled rice); processed rice products, such as rice noodles, rice chips, and rice crackers; the above-recited bakery products and confectionery products; noodles, such as pastas, buckwheat noodles, *udon* noodles, *houtou* noodles, Chinese noodles, packaged instant noodles, and instant cup noodles; other wheat processed foods; breakfast cereals or processed maize products such as corn flakes; and processed soybean products, such as *tofu,* soybean milk, soybean milk beverages, *yuba* (soybean milk skin), thin fried *tofu,* thick fried *tofu,* round fried *tofu,* soybean jam, and *miso* (soybean paste). Additionally, the fat and oil composition can be added to a variety of foods, including dairy products, such as milk, processed milk, yogurt, whey beverages, fermented lactic acid beverages, butter, and cheese; Japanese sweets, such as *yokan* (bean jelly), *uiro* (steamed rice jelly), *monaka* (wafer with bean jam), and sweet bean jam; soups, such as potage, stew, and curry; seasonings, such as soy sauce, Worcester sauce, dips, jams, and tomato ketchup; processed meat products, such as ham, sausage, and hamburgers; and processed marine products, such as steamed fish paste, baked fish paste, fried fish paste, and fish sausage.

Application of the fat and oil composition to liquid foods will then be described in detail.

The term "liquid foods" as used herein means not only liquids, such as soups and juices, but dried "liquid foods" in the form of ready-to-drink powder or solid that can be mixed with water or hot water into liquid, such as powdered soups and powdered juices.

The liquid food of the present invention can be obtained by adding the β-glucan-containing fat and oil composition to a liquid food, such as a beverage or soup. In adding, the mixture is mixed by stirring, if necessary, under heating. A conventional food emulsifier may be added to prepare an emulsion. The mixing means to be used is not particularly limited, and mixing devices, such as a mixer, can be used.

While the amount of the fat and oil composition to be added to a liquid food is not particularly limited, it is preferable to add the fat and oil composition in such an amount that the resulting liquid food may contain 0.1 to 30% by weight, particularly 0.5 to 20% by weight, of the extracted β-glucans. With an extracted β-glucan content less than 0.1% by weight, the β-glucans may fail to exhibit their functional effects. If the content exceeds 30% by weight, the extracted β-glucans can reduce the quality of the food as a whole.

In order to further suppress non-uniform distribution of the β-glucans or the liquid food containing the β-glucans due to lump formation, and the like, it is possible to add food additives or foods, such as emulsifiers, gelling agents, thickeners, and stabilizers, to the liquid food. These food additives or foods are not particularly limited as long as they are edible. Examples of the emulsifiers are lecithin, fatty acid monoglycerides, sorbitan fatty acid esters, propylene glycol fatty acid esters, sugar esters, polyoxyethylene sorbitan fatty acid esters, Tween, polyoxyethylene sorbitan trioleate, polyoxyethylene sorbitan monooleate, polyoxyethylene sorbitan monostearate, polyoxyethylene sorbitan monopalminate, and polyoxyethylene sorbitan monolaurate. Examples of the thickeners and stabilizers are pullulan, psyllium, gum arabic, gellan gum, glucomannan, guar gum, xanthan gum, tamarind gum, carrageenan, alginic acid salts, farceran, locust bean gum, pectin, curdlan, and low-molecular compounds obtained from these substances, starch, modified starch, gelatinized starch, crystalline cellulose, gelatin, dextrin, agar, and dextran. Additional useful food additives or foods include saccharides, such as glucose, fructose, sucrose, maltose, enzyme-saccharified sugar (thick malt syrup), lactose, reducing starch saccharification products, isomerized liquid sugar, sucrose-coupled malt syrup, oligosaccharides, reducing sugar polydextrose, sorbitol, reduced lactose, trehalose, xylose, xylitol, maltitol, erythritol, mannitol, fructo-oligosaccharides, soybean oligosaccharides, galacto-oligosaccharides, lactosucrose-oligosaccharides, raffinose, lactulose, palatinose-oligosaccharides, stevia, and Aspartame; stabilizers, such as phosphoric acid salts (e.g., hexametaphosphorates, secondary phosphates and primary phosphates) and alkali metal (e.g., potassium or sodium) salts of citric acid; proteins, such as whey proteins, (e.g., α-lactalbumin, β-lactoglobulin, and serum albumin), casein and other milk proteins, low-density lipoprotein, high-density lipoprotein, egg proteins (e.g., phosvitin, livetin, phosphoglycoprotein, ovalbumin, conalbumin, and ovomucoid), wheat proteins (e.g., gliadin, glutenin, prolamine, and glutelin), and other vegetable and animal proteins; protein-polysaccharide complexes, inorganic salts, such as sodium chloride, rock salt, sea salt, and potassium chloride, souring agents, such as acetic acid, lactic acid, and gluconic acid, colorants, such as β-carotin, caramel, and Monascus color, antioxidants, such as tocopherol and tea extract, eggs, such as whole eggs, egg yolk, egg white, and enzyme-processed eggs, cereals, such as bread flour, allpurpose flour, and cake flour, beans, such as soybean powder, water, flavors, dairy products, seasonings, pH adjustors, enzymes, food preservatives, shelf life extenders, fruits, fruit juices, coffee, nut pastes, spices, cacao mass, and cocoa powder. Two or more of these additives can be used in combination or in the form of a complex thereof. The amounts of the additives to be added are not particularly limited. They can be added in general amounts, for example, 0.01 to 15% by weight based on the resulting liquid food.

Examples of the liquid foods include soups, such as consomme, corn potage, egg soup, Chinese soups, stew, and curry, juices or fruit juice beverages, such as orange juice, tomato juice, banana juice, vegetable juice, fruit juices with fruit, and beverages containing fruit juice, carbonated beverages, such as cola and soda pop, milk beverages, such as milk, processed milk, yogurt, and whey drinks, seasonings, such as soy sauce, Worcester sauce, dips, jams, and tomato ketchup, coffee, cocoa, tea, green tea, woolong tea, soybean milk, fermented lactic acid beverages, alcoholic beverages, such as *sake,* whisky, brandy, beer, *sho-chu,* wines, and sparkling alcohol, vitamin rich drinks, health drinks, tonic drinks, jelly drinks, nutritious drinks, sports drinks, and coffee drinks. Also included are the above-mentioned dried liquid foods and ready-to-drink dried foods such as powdered soups which are mixed with hot water or water, etc. into liquid.

Application of the fat and oil composition to processed foods mainly comprising starch will then be described in detail.

The fat and oil composition is preferably used in an amount of 0.05 to 30% by weight, in terms of the extracted β-glucans, based on a processed food mainly comprising starch.

The processed food mainly comprising starch includes any food containing, as an ingredient, starch originated in rice, wheat, maize, potato, cassava, taro, and so forth. Examples of the processed foods include noodles, such as *udon* noodles, buckwheat noodles, Chinese noodles, chow mein noodles, sauteed *udon* noodles, spaghetti, pasta, raw noodles, dried noodles, packaged instant noodles, and instant cup noodles; cooked rices, such as doria, pilaf, beef bowl, *tempura* bowl, chicken-and-egg bowl, curry and rice, boiled rice, sterile boiled rice, and retort pouch boiled rice; processed rice products, such as rice crackers, dumplings, rice chips, and rice cakes; fried foods, such as *korokke* (Japanese style croquettes), deep-fried port cutlets, and *tempura,* and their batters; steamed buns stuffed with sweet bean jam, pork, etc.; Chinese dishes, such as shui mai, pot-stickers, spring rolls, and other Chinese snacks; Japanese pancakes, such as *okonomi-yaki* or *monja-yaki* (As-you-like-it pancakes), and octopus pancakes; and premixes for pizza, pancakes, doughnuts, *tempura,* etc. Application to the aforementioned processed foods, such as frozen foods, retorted foods, and microwave foods, is also preferred. As a matter of course, application to foods which are to be heated or thawed in a microwave oven is also preferred.

The present invention will now be illustrated in greater detail with reference to Examples, but it should be understood that the present invention is by no means limited thereto. Unless otherwise noted, all the parts and percents are by weight.

Test Example 1: Measurement of β-glucan content

Analysis of β-glucans was carried out according to the McCleary method (enzyme assay) with a β-glucan assay kit from MegaZyme. Where a sample under analysis is powder, it was sieved through a 500 μm (30 mesh) sieve, and the water content was measured. A 100 mg portion of the sample was put into each 17 ml test tube, and 200 μl of a 50% ethanol solution was added thereto to disperse the powder. Then 4 ml of a 20 mM phosphate buffer (pH 6.5) was added, and the mixture was mixed well. The mixture was heated in a boiling water bath for 1 minute. The mixture was mixed thoroughly and further heated in a hot water bath for 2 minutes. After the mixture was cooled to 50°C and allowed to stand for 5 minutes, 200 μl (10 U) of a lichenase solution was added to each tube. The lichenase solution was prepared by diluting the lichenase vial attached to the kit with 20 ml of the 20 mM phosphate buffer, and the rest of the enzyme solution was preserved in a freezer. The mixture was allowed to react at 50°C for 1 hour. Then, 5 ml of a 200 mM acetate buffer (pH 4.0) was added to each tube, followed by mixing gently. After allowing the tubes at room temperature for 5 minutes, the system was centrifuged to obtain the supernatant liquor. The supernatant liquor (100 μl) was taken into three test tubes. To one of them, 100 μl of a 50 mM acetate buffer (pH 4.0) was added, and to each of the other two was added 100 μl (0.2 U) of a β-glucosidase solution, which was prepared by diluting the glucosidase vial attached to the kit with 20 ml of the 50 mM acetate buffer. The rest of the enzyme solution was kept in a freezer. The system was allowed to react at 50°C for 10 minutes. A glucose oxidase/peroxidase solution (3 ml) was added, followed by reacting at 50°C for 20 minutes. The absorbance (EA) of each sample at 510 nm was measured. The β-glucan content was obtained from equation:

$$\beta\text{-Glucan content (w/w \%)} = (EA) \times (F/W) \times 8.46$$

where F: 100/absorbance of 100 μg glucose
W: calculated weight (mg) of anhydride

Where a sample under analysis was a liquid extract containing extracted β-glucans, the liquid was made into solid or powder as follows before analysis. To a β-glucan extract was added twice as much ethanol, and the mixture was stirred well and centrifuged. The precipitate was collected, dried well, and ground to prepare a solid β-glucan extract. After the water content of the β-glucan extract was measured, the sample was analyzed by the McCleary method (enzyme assay) using a β-glucan assay kit from MegaZyme. Each precipitate sample weighing 50 mg was put into a 17 ml test tube and dispersed in 200 μl of a 50% ethanol solution. The dispersion was analyzed in the same manner as described above.

Test Example 2: Measurement of molecular weight

The molecular weight of an extract was measured as follows. A solid extract weighing 5 mg was put into a test tube, 0.5 ml of distilled water was added thereto and heated to dissolve the extract in a boiling water bath. The solution was filtered through a 0.22 μm filter to prepare a sample for HPLC. The sample was analyzed by HPLC using a gel-filtration column, Shodex-packed column KS-805 (available from Showa Denko K.K.) at a flow rate of 0.6 ml/min at a temperature of 50°C. An RI detector was used for detection, water was used as a developing solvent, and Shodex Pullulan Standards P-82 (available from Showa Denko K.K.) was used as a molecular weight marker.

Where extracted β-glucans were a liquid extract, it was diluted with twice as much ethanol, cooled to -20°C, and allowed to stand for 1 hour to obtain a precipitate. A 5 mg portion of the precipitate was put into a tube, which was analyzed in the same manner as described above to measure the molecular weight.

Preparation Example 1: Preparation of raw material and extraction accelerator

Glutinous naked barley was polished to a polishing yield of 82%. The bran generated by this first polishing was designated bran-1. The barley having been polished to a polishing degree of 82% was further polished in a grinding type polishing machine to a polishing yield of 55%. The bran generated by the second polishing was designated grinds-1. In a 50 liter container was put 20 liters tap water and adjusted to 15°C while stirring. Six kilograms of bran-1 was added thereto and extracted while stirring for 2 hours. The mixture was centrifuged in a continuous centrifuge for solid-liquid separation, and the supernatant liquor was lyophilized to obtain 450 g of an extraction accelerator.

Preparation Example 2: Preparation of β-glucans

Into a 70 liter container was put 30 liters tap water, and 150 g of the extraction accelerator prepared in Preparation Example 1 was added. After dissolution, 7.5 kg of grinds-1 was added, followed by extraction by stirring at 50°C for 2 hours. The mixture was centrifuged for solid-liquid separation in a continuous centrifuge. The supernatant liquor separated was boiled and cooled to give 15 liters of a slightly viscous β-glucan solution (sample 1). As a result of analysis according to Test Example 1, the β-glucan content was found to be 3%. As a result of analysis according to Test Example 2, the extract was detected in the molecular weight range of from 10,000 to 90,000, with the maximum peak at 40,000. The maximum peak was confirmed to be β-glucans by the method of Test Example 1.

Preparation Example 3: Preparation of β-glucans

To a β-glucan solution prepared in the same manner as in Preparation Example 2 was added twice as much ethanol. A precipitate thus formed was collected and dried to give 460 g of a β-glucan extract (sample 2), which was found to have a β-glucan purity of 91% as a result of analysis according to Test Example 1. As a result of analysis according to Test Example 2, the extract was detected in the molecular weight range of from 10,000 to 200,000, with the maximum peak at 40,000. The maximum peak was confirmed to be β-glucans by the method of Test Example 1.

Preparation Example 4: Preparation of β-glucans

A β-glucan solution prepared in the same manner as in Preparation Example 2 was lyophilized as such to give a β-glucan extract (sample 3) weighing 580 g. Sample 3 was found to have a β-glucan purity of 76% as a result of analysis according to Test Example 1. As a result of analysis according to Test Example 2, the extract was detected in the molecular weight range of from 10,000 to 200,000, with the maximum peak at 40,000. The maximum peak was confirmed to be β-glucans by the method of Test Example 1.

Preparation Example 5: Preparation of β-glucans

Into a 70 liter container was put 30 liters tap water, and 60 g of sodium hydroxide was added thereto while stirring. After dissolution, 7 kg of grinds-1 was added, followed by extraction by stirring at 30°C for 2 hours. The mixture was neutralized with hydrochloric acid and separated into solid and liquid by means of a continuous centrifuge. The supernatant liquor separated was boiled to give 15 liters of a slightly viscous β-glucan solution (sample 4). As a result of analysis according to Test Example 1, the β-glucan content was found to be 1.8 wt%. As a result of analysis according to Test Example 2, the extract showed no peak in a molecular weight range between 3000 and 100,000 but an extremely broad peak in the range of from 100,000 to 500,000. The fraction collected in the molecular weight range of 100,000 or greater was confirmed to be β-glucans by the method of Test Example 1.

Method of evaluation in Examples 1 to 32 and Comparative Examples 1 to 23:

In Examples 1 to 32 and Comparative Examples 1 to 23 hereinafter described, evaluation was made in terms of stability and texture (smoothness, hardness, and flavor) according to necessity. Stability was evaluated by visually inspecting any change in appearance after storage at 5°C for 1 month. Texture was organoleptically evaluated by 10 panel members and rated A to C according to standards shown below. The rating given to a sample by the greatest number out of 10 panel members was the rating of the sample. The results obtained are shown in Tables 1 and 2, in which marks "-" indicate no evaluation made.

Standards of evaluation:

1) Stability

A: Excellent in stability.
B: Change in appearance, such as slight phase separation, observed.
C: Phase separation observed.

2) Texture
2-1) Smoothness

A: Very smooth
B: Smooth
C: Not smooth

2-2) Hardness

A: Very soft
B: Soft
C: Not soft

2-3) Flavor

A: Superior
B: Slightly inferior
C: Inferior

Example 1: Fat and oil composition containing β-glucans extracted from gramineous plant

A hundred parts of sample 2 obtained in Preparation Example 3 and 100 parts of soybean oil were thoroughly mixed in a kneader. The mixture was kept at 60°C for 10 minutes and then cooled to room temperature, whereupon it became creamy to give a fat and oil composition-1 containing β-glucans extracted from a gramineous plant according to the present invention (β-glucan content: 45.50%). The β-glucans were found uniformly dispersed.

Example 2: Fat and oil composition containing β-glucans extracted from gramineous plant

Three hundred parts of sample 3 obtained in Preparation Example 4 was mixed with 100 parts of palm oil melted by heating at 70°C and 1 part of lecithin in a high-speed homomixer. The mixture was left to stand at 50°C for 20 minutes and cooled to room temperature to give a lumpy fat and oil composition-2 containing β-glucans extracted from a gramineous plant according to the present invention (β-glucan content: 56.90%). The β-glucans were found uniformly dispersed.

Example 3: Fat and oil composition containing β-glucans extracted from gramineous plant

Fifty parts of sample 3 obtained in Preparation Example 4 was mixed with 30 parts of palm olein oil, 70 parts of rapeseed oil, and 0.2 part of protease-hydrolyzed egg yolk in a mixer. The mixture was left to stand at 65°C for 15 minutes and cooled to room temperature to give a creamy fat and oil composition-3 containing β-glucans extracted from a gramineous plant according to the present invention (β-glucan content: 25.30%). The β-glucans were found uniformly dispersed.

Example 4: Fat and oil composition containing β-glucans extracted from gramineous plant

Five parts of sample 2 obtained in Preparation Example 3 was mixed with 40 parts of rice oil, 20 parts of olive oil, and 35 parts of safflower oil in a high-speed homomixer. The mixture was allowed to stand at 50°C for 30 minutes and cooled to room temperature to give a fat and oil composition-4 containing β-glucans extracted from a gramineous plant according to the present invention (β-glucan content: 4.60%), which had almost the same viscosity as the starting oils but showed slight turbidity. The β-glucans were found uniformly dispersed.

Example 5: Fat and oil composition containing β-glucans extracted from gramineous plant

Thirteen parts of sample 1 obtained in Preparation Example 2 was mixed with 20 parts of hydrogenated soybean oil (melting point: 45°C), 35 parts of palm oil, 30 parts of cotton seed oil, and 0.2 part of soybean lysolecithin. The mixture was allowed to stand at 70°C for 10 minutes and then emulsified in a high-speed mixer, followed by rapidly cooling for plasticization to give a fat and oil composition-5 containing β-glucans extracted from a gramineous plant according to the present invention (β-glucan content: 0.40%), which had margarine-like physical properties. The β-glucans were found uniformly dispersed.

Example 6: Fat and oil composition containing β-glucans extracted from gramineous plant

Fifty parts of sample 4 obtained in Preparation Example 5 was mixed with 27.6 parts of hydrogenated fish oil (melting point: 36°C), 18 parts of corn salad oil, and 0.4 part of glycerol monotartrate by stirring at 50°C for 30 minutes. The mixture was emulsified in a high-speed mixer, followed by rapidly cooling for plasticization to give a fat and oil composition-6 containing β-glucans extracted from a gramineous plant according to the present invention (β-glucan content: 0.94%), which had fat spread-like physical properties. The β-glucans were found uniformly dispersed.

Example 7: Fat and oil composition containing β-glucans extracted from gramineous plant

Twenty parts of sample 1 obtained in Preparation Example 2 was mixed with 0.3 part of olive oil (melting point: 36°C) and 0.1 part of casein sodium. The mixture was allowed to stand at 55°C for 15 minutes, emulsified in a high-speed mixer, and spray-dried to give a powdered fat and oil composition-7 containing β-glucans extracted from a gramineous plant according to the present invention (β-glucan content: 60.00%). The β-glucans were found uniformly dispersed.

Example 8: Production of shortening

An oil phase consisting of 30 parts of palm oil, 50 parts of hydrogenated palm oil, 20 parts of rapeseed oil, and 0.3 part of lecithin was melted at 70°C. To 100 parts of the oil phase was added 5.0 parts of sample 2 obtained in Preparation Example 3, and the mixture was allowed to stand at 70°C for 30 minutes. The mixture was then stirred in a homomixer at a high rotational speed for 2 minutes to give a fat and oil composition-8 containing β-glucans extracted from a gramineous plant according to the present invention. The β-glucans were found by visual observation sufficiently dispersed in the fat and oil. The composition was rapidly cooled for plasticization and cooled to 5°C to obtain shortening (β-glucan content: 4.30%) according to the present invention. The shortening of the present invention was evaluated for smoothness and flavor. The results obtained are shown in Table 1. It is seen that the resulting shortening is superior to Comparative Example 1 described hereunder in smoothness and flavor. Although the step of crystal aging had been omitted, the shortening of the present invention can be said to have enjoyed the effects of forming moderate crystals with excellent texture, accelerating such crystallization, and preventing reduction of flavor that might have been caused by an emulsifier.

Comparative Example 1: Production of shortening

An oil phase consisting of 30 parts of palm oil, 50 parts of hydrogenated palm oil, 20 parts of rapeseed oil, and 0.3 part of lecithin was melted at 70°C, stirred in a homomixer at a high rotational speed for 2 minutes, rapidly cooled for plasticization, and cooled to 5°C to obtain an edible fat and oil composition. The resulting shortening was evaluated for smoothness and flavor for comparison. The results obtained are shown in Table 2, which prove the resulting shortening to be much inferior in flavor.

Example 9: Production of margarine

A hundred part of an edible fat and oil consisting of palm oil, hydrogenated palm oil, rapeseed oil, and sorbitan fatty acid esters at a weight ratio of 30:50:20:0.3 was melted at 70°C. Eight parts of sample 3 obtained in Preparation Example 4 was added thereto, and the mixture was left to stand at 65°C for 30 minutes. A solution of 0.5 part of defatted milk powder and 1 part of edible salt in 16 parts of hot water (70°C) was slowly added to the mixture and mixed therewith while stirring in a homomixer. Then, the mixture was rapidly cooled to plasticize, maintained at 25°C overnight, and cooled to 5°C to give margarine according to the present invention (β-glucan content: 4.80%). The β-glucans were found uniformly dispersed. The margarine of the present invention was evaluated for stability, smoothness, and flavor. The results are shown in Table 1. The resulting margarine had a fine and smooth texture. Further, the margarine was more flavorful than that of Comparative Example 2 described hereunder, proving the effect of suppressing a reduction in flavor which might have been caused by the emulsifier.

Comparative Example 2: Production of margarine

A hundred part of an edible fat and oil consisting of palm oil, hydrogenated palm oil, rapeseed oil, and sorbitan fatty acid esters at a weight ratio of 30:50:20:0.3 was melted at 70°C. To the mixture was slowly added a solution of 0.5 part of defatted milk powder and 1 part of edible salt in 16 parts of hot water (70°C) while stirring in a homomixer. After mixing, the mixture was rapidly cooled to plasticize, maintained at 25°C overnight, and cooled to 5°C to give margarine. The resulting margarine was evaluated for stability, smoothness, and flavor for comparison. The results are shown in Table 2.

Example 10: Production of dressing

Ten parts of sample 2 prepared in Preparation Example 3, 10 parts of egg yolk, 1.5 parts of edible salt, 11 parts of vinegar, 2.5 parts of soft white sugar, 0.05 part of mustard powder, and 0.05 part of onion powder were mixed by stirring in a mixer at a high speed for 5 minutes to prepare an aqueous phase. While the aqueous phase was further stirred in a homomixer at a high speed, 75 parts of soybean salad oil heated to 70°C was slowly added thereto and mixed. The mixture was left to stand at 50°C for 10 minutes, emulsified, and cooled at 5°C for 24 hours to obtain a dressing (β-glucan content: 8.27%) according to the present invention. The β-glucans were found uniformly dispersed. The dressing of the present invention was evaluated for stability and flavor. The results are shown in Table 1. The resulting dressing was proved excellent in stability and flavor.

Comparative Example 3: Production of dressing

Ten parts of egg yolk, 1.5 parts of edible salt, 11 parts of vinegar, 2.5 parts of soft white sugar, 0.05 part of mustard powder, and 0.05 part of onion powder were mixed by stirring in a mixer at a high speed for 5 minutes to prepare an aqueous phase. A dressing was prepared by using the aqueous phase in the same manner as in Example 10. The resulting dressing was evaluated for stability and flavor for comparison. The results are shown in Table 2.

Example 11: Production of dressing

Ten parts of egg yolk, 1.5 parts of edible salt, 11 parts of vinegar, 2.5 parts of soft white sugar, 0.05 part of mustard powder, and 0.05 part of onion powder were mixed by stirring in a mixer at a high speed for 5 minutes to prepare an aqueous phase. While the aqueous phase was further stirred in a homomixer at a high speed, 75 parts of the fat and oil composition-4 containing β-glucans extracted from a gramineous plant which was prepared in Example 4 was slowly added thereto. The mixture was emulsified and cooled at 5°C for 24 hours to give a dressing (β-glucan content: 3.45%) according to the present invention. The β-glucans were found uniformly dispersed. The dressing of the present invention was evaluated for stability and flavor. The results are shown in Table 1. The resulting dressing was proved excellent in stability and flavor.

Comparative Example 4: Production of dressing

Ten parts of egg yolk, 1.5 parts of edible salt, 11 parts of vinegar, 2.5 parts of soft white sugar, 0.05 part of mustard powder, and 0.05 part of onion powder were mixed by stirring in a mixer at a high speed for 5 minutes to prepare an aqueous phase. While the aqueous phase was further stirred in a homomixer at a high speed, 75 parts of a fat and oil mixture consisting of 40 parts of rice oil, 20 parts of olive oil, and 35 parts of safflower oil was slowly added thereto. The mixture was emulsified and cooled at 5°C for 24 hours to give a dressing, which was evaluated for stability and flavor for comparison. The results are shown in Table 2.

Example 12: Production of mayonnaise

Thirty parts of soybean salad oil was added to 30 parts of sample 1 obtained in Preparation Example 2, and the mixture was stirred for preliminary emulsification to prepare a fat and oil composition containing β-glucans extracted from a gramineous plant according to the present invention. A thoroughly stirred mixture consisting of 9 parts of egg yolk, 5.2 parts of starch, 8.2 parts of sugar, 2.8 parts of edible salt, 8 parts of vinegar, 1 part of seasoning spices, and 6 parts of water was added to the fat and oil composition, and the mixture was finally emulsified in a colloid mill to give mayonnaise (β-glucan content: 0.09%) according to the present invention. The β-glucans were found uniformly dispersed. The mayonnaise of the present invention was evaluated for stability, smoothness, and flavor. The results obtained are shown in Table 1. The resulting mayonnaise underwent no separation of water when stored for 1 month and had a smooth texture and a very good flavor.

Comparative Example 5: Production of mayonnaise

Thirty parts of soybean salad oil was added to 30 parts of water, and the mixture was stirred for preliminary emulsification to prepare a fat and oil composition. A thoroughly stirred mixture consisting of 9 parts of egg yolk, 5.2 parts of starch, 8.2 parts of sugar, 2.8 parts of edible salt, 8 parts of vinegar, 1 part of seasoning spices, and 6 parts of water was added to the fat and oil composition, and the mixture was finally emulsified in a colloid mill to prepare mayonnaise, which was evaluated for stability, smoothness and flavor for comparison. The results obtained are shown in Table 2.

Example 13: Production of mayonnaise

Nine parts of egg yolk, 8.2 parts of sugar, 2.8 parts of edible salt, 8 parts of vinegar, 1 part of seasoning spices, and 36 parts of sample 4 obtained in Preparation Example 5 were mixed to prepare an aqueous phase. To the aqueous phase were added 25 parts of rapeseed oil and 10 parts of the fat and oil composition of Example 1, and the mixture was preliminarily emulsified by stirring and finally emulsified in a colloid mill to prepare mayonnaise (β-glucan content: 5.20%) according to the present invention. The β-glucans were found uniformly dispersed. The mayonnaise of the present invention was evaluated for stability, smoothness, and flavor. The results obtained are shown in Table 1. The resulting mayonnaise underwent no separation of water when stored for 1 month and had a smooth texture and a very good flavor.

Comparative Example 6: Production of mayonnaise

Nine parts of egg yolk, 8.2 parts of sugar, 2.8 parts of edible salt, 8 parts of vinegar, 1 part of seasoning spices, and 36 parts of water were mixed to prepare an aqueous phase. To the aqueous phase were added 25 parts of rapeseed oil and 10 parts of palm oil, and the mixture was preliminarily emulsified by stirring and finally emulsified in a colloid mill to prepare mayonnaise, which was evaluated for stability, smoothness, and flavor for comparison. The results obtained are shown in Table 2.

Example 14: Production of fat spread

A mixture consisting of 27.6 parts of hydrogenated fish oil (melting point: 36°C), 18.4 parts of cotton seed oil, 40 parts of sample 1 obtained in Preparation Example 2, 12.3 parts of water, 1 part of edible salt, 0.5 part of defatted milk powder, 0.2 part of flavors, and 0.3 part of lecithin was emulsified and rapidly cooled for plasticization to prepare fat spread (β-glucan content: 1.20%) according to the present invention. The β-glucans were found uniformly dispersed. The fat spread of the present invention was evaluated for stability, smoothness, and flavor. The results are shown in Table 1. The resulting fat spread underwent no separation of water when stored for 1 month and had a smooth texture and a very good flavor.

Comparative Example 7: Preparation of fat spread

A mixture consisting of 27.6 parts of hydrogenated fish oil (melting point: 36°C), 18.4 parts of cotton seed oil, 52.3 parts of water, 1 part of edible salt, 0.5 part of defatted milk powder, 0.2 part of flavors, and 0.3 part of lecithin was emulsified and rapidly cooled for plasticization to prepare fat spread, which was evaluated for stability, smoothness and flavor for comparison. The results are shown in Table 2.

Example 15: Production of curry sauce mix

Forty-four parts of wheat flour (cake flour) and 34 parts of the shortening obtained in Example 8 were pan-fried brown, and the resulting roux was mixed with 8 parts of a commercially available curry powder mix to obtain a curry sauce mix (β-glucan content: 1.70%) according to the present invention. The β-glucans were found uniformly dispersed.

Example 16: Production of cookies

Fifty parts of the fat and oil composition-3 containing β-glucans extracted from a gramineous plant obtained in Example 3 and 50 parts of soft white sugar were kneaded into cream in a hobart mixer at a high speed for 6 minutes.

A mixture of 15 parts net of whole eggs, 1 part of edible salt, and 0.5 part of ammonium hydrogencarbonate was added thereto, followed by mixing at a medium speed for 30 seconds. A hundred parts of sieved wheat flour was added, followed by mixing at a low speed for 30 seconds to prepare dough. The dough was put into a cylinder of 6 cm in diameter, pressed out by 1 cm, and cut. The cut pieces of the dough were baked at 200°C for 13 minutes to obtain cookies (β-glucan content: 5.86%) according to the present invention. The β-glucans were found uniformly dispersed. The cookies of the present invention were evaluated for hardness and flavor. The results are shown in Table 1.

Comparative Example 8: Production of cookies

Fifty parts of a fat and oil mixture consisting of 30 parts of palm olein oil, 70 parts of rapeseed oil, and 0.2 part of protease-hydrolyzed egg yolk and 50 parts of soft white sugar were kneaded into cream in a hobart mixer at a high speed for 6 minutes. A mixture of 15 parts net of whole eggs, 1 part of edible salt, and 0.5 part of ammonium hydrogencarbonate was added thereto, followed by mixing at a medium speed for 30 seconds. The mixture was further processed in the same manner as in Example 16 to obtain cookies, which were evaluated for hardness and flavor for comparison. The results are shown in Table 2.

Example 17: Production of cookies

Fifty parts of the fat and oil composition-5 containing β-glucans extracted from a gramineous plant obtained in Example 5 and 40 parts of soft white sugar of beet were kneaded into cream in a hobart mixer at a high speed for 6 minutes. Twenty parts of raisin paste was added and mixed into the cream at a medium speed for 30 seconds. Sieved foxtail millet flour was added, followed by mixing at a low speed for 30 seconds to prepare dough. The dough was put into a cylinder of 6 cm in diameter, pressed out by 1 cm, and cut. The cut pieces of the dough were baked at 160°C for 15 minutes to obtain cookies (β-glucan content: 0.10%) according to the present invention. The β-glucans were found uniformly dispersed. The cookies of the present invention were evaluated for hardness and flavor. The results are shown in Table 1. The resulting cookies had a satisfactory texture despite the fact that neither eggs nor dairy products was used.

Comparative Example 9: Production of cookies

A mixture of 20 parts of hydrogenated soybean oil (melting point: 45°C), 35 parts of palm oil, 30 parts of cotton seed oil, and 0.2 part of soybean lysolecithin was allowed to stand at 70°C for 10 minutes and then emulsified in a high-speed mixer, followed by rapid cooling for plasticization to prepare an edible fat and oil composition having margarine-like physical properties. Fifty parts of the fat and oil composition and 40 parts of soft white sugar of beet were kneaded into cream in a hobart mixer at a high speed for 6 minutes. Twenty parts of raisin paste was added, followed by mixing at a medium speed for 30 seconds. The resulting mixture was further processed in the same manner as in Example 17 to obtain cookies, which were evaluated for hardness and flavor for comparison. The results are shown in Table 2.

Example 18: Production of chocolate

Twelve parts of cacao mass, 45 parts of powdered sugar, 20 parts of whole milk powder, 13 out of 23 parts of cacao butter, and 2 parts of sample 2 of Preparation Example 3 were put into a hobart mixer and mixed with a beater at a medium speed for 3 minutes. The mixture was rolled and conched to prepare a fat and oil composition containing β-glucans extracted from a gramineous plant according to the present invention. As observed with the naked eye, the β-glucans were found uniformly dispersed. The rest of cacao butter was added thereto and mixed to obtain chocolate mass. The chocolate mass was subjected to tempering, poured into a mold, and cooled down to obtain chocolate of the present invention, which was evaluated for smoothness, hardness, and flavor. The results obtained are shown in Table 1. The resulting chocolate had good melt in the mouth and a good flavor.

Comparative Example 10: Production of chocolate

Twelve parts of cacao mass, 45 parts of powdered sugar, 20 parts of whole milk powder, 13 out of 23 parts of cacao butter, and 2 parts of a fat and oil composition consisting of 30 parts of palm olein oil, 70 parts of rapeseed oil, and 0.2 part of protease-processed egg yolk were put into a hobart mixer and mixed with a beater at a medium speed for 3 minutes. The mixture was rolled and conched to prepare a fat and oil composition. The rest of cacao butter was added thereto and mixed to obtain chocolate mass. The chocolate mass was further processed in the same manner as in Example 18 to obtain chocolate, which was evaluated for smoothness, hardness, and flavor for comparison. The results obtained are shown in Table 2.

Example 19: Production of chocolate

Twelve parts of cacao mass, 45 parts of powdered sugar, 20 parts of whole milk powder, 23 parts of cacao butter, and 20 parts of the fat and oil composition-2 containing β-glucans extracted from a gramineous plant prepared in Example 2 were put into a hobart mixer and mixed with a beater at a medium speed for 3 minutes. The mixture was rolled and conched to obtain chocolate mass. The chocolate mass was subjected to tempering, poured into a mold, and cooled down to obtain solid chocolate of the present invention. The β-glucans were found uniformly dispersed. The chocolate of the present invention was evaluated for smoothness, hardness, and flavor. The results are shown in Table 1. The resulting chocolate had good melt in the mouth and a good flavor.

Comparative Example 11: Production of chocolate

Twelve parts of cacao mass, 45 parts of powdered sugar, 20 parts of whole milk powder, 23 parts of cacao butter, and 20 parts of palm oil were put into a hobart mixer. The same procedures of Example 19 were then followed to obtain a chocolate product, which was evaluated for smoothness, hardness, and flavor for comparison. The results obtained are shown in Table 2.

Example 20: Production of bread

Bread was made by using the β-glucan-containing margarine obtained in Example 9. A hundred parts of wheat flour, 3 parts of yeast, 4 parts of sugar, 2 parts of edible salt, 6 parts of the margarine obtained in Example 9, and 60 parts of water were mixed in a hopper mixer at a mixing temperature of 28°C at a low speed for 2 minutes and at a high speed for 4 minutes to prepare bread dough. The dough was allowed to ferment at 28°C for 60 minutes and divided into 450 g portions, which were each formed into a ball and allowed to prove at 28°C for 20 minutes. The dough was passed through a sheeter three times, shaped, put into a one-loaf pan, and finally proofed at 38°C and 90% RH until it rose 2 cm above the lip of the pan. The final proofing took 42 minutes. The proofed dough was baked at 220°C for 23 minutes to obtain a loaf of bread (β-glucan content: 2.10%) according to the present invention. The β-glucans were found uniformly dispersed. The bread of the present invention was evaluated for hardness and flavor. The results obtained are shown in Table 1. The resulting bread had softness, good volume and a satisfactory texture.

Comparative Example 12: Production of bread

Bread was made in the same manner as in Example 20, except for replacing the margarine used in Example 20 with margarine which was prepared in the same manner as in Example 9 except for using no β-glucans. The resulting bread was evaluated for hardness and flavor for comparison. The results obtained are shown in Table 2.

Example 21: Production of bread

A hundred parts of wheat flour, 3 parts of yeast, 4 parts of sugar, 2 parts of edible salt, 2 parts of the powdered fat and oil obtained in Example 7, 4 parts of shortening, 50 parts of the sample obtained in Preparation Example 2, and 13 parts of water were mixed in a hopper mixer at a mixing temperature of 28°C at a low speed for 2 minutes and at a high speed for 4 minutes to prepare bread dough. The dough was allowed to ferment at 28°C for 60 minutes and divided into 450 g portions, which were each formed into a ball and allowed to prove at 28°C for 20 minutes. The dough was passed through a sheeter three times, shaped, put into a one-loaf pan, and finally proofed at 38°C and 90% RH until it rose 2 cm above the lip of the pan. The final proofing took 46 minutes. The proofed dough was baked at 210°C for 30 minutes to obtain a loaf of bread (β-glucan content: 2.00%) according to the present invention. The β-glucans were found uniformly dispersed. The bread of the present invention was evaluated for hardness and flavor. The results obtained are shown in Table 1. The resulting bread had softness, good volume and a satisfactory texture.

Comparative Example 13: Production of bread

A hundred parts of wheat flour, 3 parts of yeast, 4 parts of sugar, 2 parts of edible salt, 2 parts of a powdered fat and oil prepared in the same manner as in Example 7 except for using no β-glucans, 4 parts of shortening, and 63 parts of water were mixed in a hopper mixer at a mixing temperature of 28°C at a low speed for 2 minutes and at a high speed for 4 minutes to prepare bread dough. The dough was further processed in the same manner as in Example 21 to obtain a loaf of bread, which was evaluated for hardness and flavor for comparison. The results are shown in Table 2.

Example 22: Making of boiled rice

Japonica rice cultivar Koshihikari made in Niigata was washed well with water. To 100 parts of washed rice were added 60 parts of water and 4 parts of the fat and oil composition-1 containing β-glucans extracted from a gramineous plant which was obtained in Example 1. The rice was boiled in an electric rice cooker to obtain boiled rice (β-glucan content: 1.10%). The β-glucans were found uniformly dispersed. The boiled rice was evaluated for hardness. The results are shown in Table 1. The resulting boiled rice had a light and soft texture.

Comparative Example 14: Making of boiled rice

Japonica rice cultivar Koshihikari made in Niigata was washed well with water. To 100 parts of washed rice were added 60 parts of water and 4 parts of soybean oil. The rice was boiled in an electric rice cooker to obtain boiled rice, which was evaluated for hardness for comparison. The results are shown in Table 2.

Example 23: Production of popcorn

Into a pan were put 100 parts of popcorn kernels, 2 parts of edible salt, and 10 parts of the fat and oil composition-4 containing β-glucans extracted from a gramineous plant obtained in Example 4, and the pot covered with a lid was heated on fire to obtain popcorn (β-glucan content: 0.41%). The β-glucans were found uniformly dispersed. The popcorn of the present invention was evaluated for texture. The results are shown in Table 1. The resulting popcorn had good quality with a smooth and light texture.

Example 24: Production of *tofu*

*Tofu* was produced by using the shortening prepared in Example 8. Soybeans were soaked in water. A hundred parts of soaked soybeans was ground together with 140 parts of water, boiled at 100°C for 5 minutes, put into a cotton bag, and squeezed to obtain soy milk. To the soy milk were added 3 parts of a coagulant (calcium sulfate) and 10 parts of the shortening obtained in Example 8. The mixture was gently stirred, coagulated at 75°C, and poured into a strainer lined with cotton cloth, left to stand for 30 minutes to obtain *tofu* (β-glucan content: 1.70%) according to the present invention. The β-glucans were found uniformly dispersed. The resulting *tofu* was evaluated for smoothness and flavor. The results are shown in Table 1. The resulting *tofu* had a good texture.

Comparative Example 15: Production of *tofu*

Soybeans were soaked in water. A hundred parts of soaked soybeans was ground together with 140 parts of water, boiled at 100°C for 5 minutes, put into a cotton bag, and squeezed to obtain soy milk. To the soy milk were added 3 parts of a coagulant (calcium sulfate) and 10 parts of shortening prepared in the same manner as in Example 8 except for using no β-glucans. The mixture was gently stirred, coagulated at 75°C, and poured into a strainer lined with cotton cloth, left to stand for 30 minutes to obtain *tofu,* which was evaluated for smoothness and flavor for comparison. The results are shown in Table 2.

Example 25: Production of soft chocolate

A mixture consisting of 50 parts of sugar, 5 parts of cacao mass, 15 parts of whole fat milk powder, 30 parts of the fat and oil composition-2 containing β-glucans extracted from a gramineous plant which was obtained in Example 2, 0.3 part of lecithin, and 0.04 part of vanillin was subjected to rolling and conching in a usual manner to obtain soft chocolate (β-glucan content: 17%) according to the present invention. The β-glucans were found uniformly dispersed. The soft chocolate of the present invention was evaluated for smoothness, hardness, and flavor. The results obtained are shown in Table 1. The resulting soft chocolate underwent no blooming and had a good flavor.

Comparative Example 16: Production of soft chocolate

A mixture consisting of 50 parts of sugar, 5 parts of cacao mass, 15 parts of whole fat milk powder, 30 parts of palm oil, 0.3 part of lecithin, and 0.04 part of vanillin was subjected to rolling and conching in a usual manner to obtain soft chocolate, which was evaluated for smoothness, hardness, and flavor for comparison. The results obtained are shown in Table 2.

22

Example 26: Production of water-free cream

Thirty-five parts of the fat and oil composition-8 containing β-glucans extracted from a gramineous plant which was obtained in Example 8, 45 parts of sugar, 10 parts of a tasty powder, and 10 parts of milk powder were mixed to obtain water-free cream (β-glucan content: 1.50%) according to the present invention. The β-glucans were found uniformly dispersed. The water-free cream of the present invention was evaluated for smoothness and flavor. The results are shown in Table 1. The resulting water-free cream had good melt in the mouth and a very good flavor.

Comparative Example 17: Production of water-free cream

Thirty-five parts of a fat and oil composition prepared in the same manner as in Example 8 except for using no β-glucans, 45 parts of sugar, 10 parts of a tasty powder, and 10 parts of milk powder were mixed to obtain water-free cream, which was evaluated for smoothness and flavor for comparison. The results are shown in Table 2.

Example 27: Production of whipped cream for cream sandwiches

A mixture of 100 parts of the fat and oil composition-8 containing β-glucans extracted from a gramineous plant which was prepared in Example 8 and 0.1 part of a monoglyceride was beaten into whipped cream having a specific gravity of 0.3. A hundred parts of sugar syrup was added, and the cream was further beaten to prepare whipped cream having a specific gravity of 0.65 β-glucan content: 2.15%), which was for cream sandwiches. The β-glucans were found uniformly dispersed. The whipped cream of the present invention was evaluated for smoothness and flavor. The results are shown in Table 1. The resulting whipped cream had a very good flavor.

Comparative Example 18: Production of whipped cream for cream sandwiches

Whipped cream for cream sandwiches was made in the same manner as in Example 27, except for using 100 parts of a fat and oil composition prepared in the same manner as in Example 8 except for using no β-glucans. The resulting whipped cream was evaluated for smoothness and flavor for comparison. The results obtained are shown in Table 2.

Example 28: Production of hard candy

Thirty-five parts of a fat and oil composition consisting of 100 parts of the fat and oil of Example 1, 100 parts of the fat and oil of Example 2, 23 parts of a polyglycerol fatty acid ester, 14 parts of a glycerol fatty acid ester, and 4 parts of a sucrose fatty acid ester, 35 parts of sugar, 8.5 parts of thick malt syrup, 1.5 parts of defatted milk powder, and 40 parts of water were mixed into an oil-in-water emulsion, which was boiled down until the temperature reached 140°C and further concentrated until the water content was reduced to 1.9%. The resulting thick syrup was cooled and molded to obtain hard candy (β-glucan content: 17.80%). The β-glucans were found uniformly dispersed. The hard candy of the present invention was evaluated for smoothness and flavor. The results are shown in Table 1. The resulting hard candy underwent no bleeding of oily components during storage and had a good flavor.

Comparative Example 19: Production of hard candy

Thirty-five parts of a fat and oil composition consisting of 100 parts of soybean oil, 100 parts of palm oil, 23 parts of a polyglycerol fatty acid ester, 14 parts of a glycerol fatty acid ester, and 4 parts of a sucrose fatty acid ester, 35 parts of sugar, 8.5 parts of thick malt syrup, 1.5 parts of defatted milk powder, and 40 parts of water were mixed into an oil-in-water emulsion, which was boiled down until the temperature reached 140°C and further concentrated to water content of 1.9%. The resulting thick syrup was cooled and molded to obtain hard candy. The resulting hard candy was evaluated for smoothness and flavor for comparison. The results are shown in Table 2.

Example 29: Production of whipped cream

In 50 parts of water heated to 60°C were dissolved 5 parts of defatted milk powder and 0.1 part of sodium tripolyphosphate while stirring to prepare an aqueous phase. Separately, 10 parts of the fat and oil composition of Example 1, 20 parts of the fat and oil composition of Example 2, and 15 parts of the fat and oil composition of Example 3 were mixed to prepare an oily phase. The oily phase was mixed with the aqueous phase by stirring to prepare a preliminary emulsion. The preliminary emulsion was homogenized under a pressure of 5 MPa, sterilized in a VTIS sterilization apparatus at 142°C for 4 seconds, re-homogenized under a pressure of 5 MPa, cooled to 5°C, and then aged in a refrigerator for 24 hours to give whipped cream (β-glucan content: 19.73%) according to the present invention. The β-glucans were found uniformly dispersed. The whipped cream of the present invention was evaluated for stability, smoothness, and flavor. The results are shown in Table 1. The resulting whipped cream was proved to have satisfactory qualities of overrun, emulsion stability, heat resistant shape retention, flavor, melt-in-the-mouth, and shapability in piping into rosettes.

Comparative Example 20: Production of whipped cream

In 50 parts of water heated to 60°C were dissolved 5 parts of defatted milk powder and 0.1 part of sodium tripolyphosphate while stirring to prepare an aqueous phase. Separately, 10 parts of soybean oil, 20 parts of palm oil, and 15 parts of rapeseed oil were mixed to prepare an oily phase. The oily phase was mixed with the aqueous phase by stirring to prepare a preliminary emulsion. The preliminary emulsion was further processed in the same manner as in Example 29 to obtain whipped cream, which was evaluated for stability, smoothness, and flavor for comparison. The results obtained are shown in Table 2.

Example 30: Production of milk substitute composition

In 64 parts of water heated to 60°C were dissolved 25 parts of defatted milk powder, 0.2 part of sodium hexametaphosphate, 0.2 part of sodium citrate, and 0.3 part of a sucrose fatty acid ester while stirring to prepare an aqueous phase. To the aqueous phase was added 10 parts of the fat and oil composition of Example 6, 0.3 part of a glycerol fatty acid ester and mixed by stirring to prepare a preliminary emulsion. The preliminary emulsion was homogenized under a pressure of 5 MPa, sterilized in a VTIS sterilization apparatus at 142°C for 4 seconds, re-homogenized under a pressure of 15 MPa, and cooled to 5°C to obtain a milk substitute composition (β-glucan content: 0.09%) according to the present invention. The β-glucans were found uniformly dispersed. The milk substitute composition of the present invention was evaluated for stability and flavor. The results are shown in Table 1. The resulting milk substitute composition was proved satisfactory in both flavor and emulsion stability.

Comparative Example 21: Production of milk substitute composition

In 64 parts of water heated to 60°C were dissolved 25 parts of defatted milk powder, 0.2 part of sodium hexametaphosphate, 0.2 part of sodium citrate, and 0.3 part of a sucrose fatty acid ester while stirring to prepare an aqueous phase. To the aqueous phase were added 10 parts of the fat and oil composition of Comparative Example 6 and 0.3 part of a glycerol fatty acid ester and mixed by stirring to prepare a preliminary emulsion. The preliminary emulsion was further processed in the same manner as in Example 30 to give a milk substitute composition. The resulting milk substitute composition was evaluated for stability and flavor for comparison. The results are shown in Table 2.

Example 31: Production of food (margarine) prophylactic for habitual disease

Ten parts of hydrogenated soybean oil (melting point: 45°C), 35 parts of palm oil, 10 parts of the fat and oil composition-1 containing β-glucans extracted from a gramineous plant which was obtained in example 1, 30 parts of an interesterified oil containing 10% or more of plant sterol or plant sterol fatty acid esters, 13.3 parts of sample 1 obtained in Preparation Example 2, 1 part of edible salt, 0.5 part of defatted milk powder, and 0.2 part of flavor were emulsified and rapidly cooled for plasticization to produce margarine having a cholesterol lowering effect (β-glucan content: 4.95%) according to the present invention. The β-glucans were found uniformly dispersed. The margarine having a cholesterol lowering effect according to the present invention was evaluated for smoothness and flavor. The results are shown in Table 1. The resulting margarine had good melt in the mouth and a good flavor.

Comparative Example 22: Production of food (margarine) prophylactic for habitual disease

Ten parts of hydrogenated soybean oil (melting point: 45°C), 35 parts of palm oil, 10 parts of soybean oil, 30 parts of an interesterified oil containing 10% or more of plant sterol or plant sterol fatty acid esters, 13.3 parts of water, 1 part of edible salt, 0.5 part of defatted milk powder, and 0.2 part of flavor were emulsified and rapidly cooled for plasticization to produce margarine having a cholesterol lowering effect, which was evaluated for smoothness and flavor for comparison. The results are shown in Table 2.

Example 32: Production of drug prophylactic for habitual diseases

Three parts of high purity DHA (purity: 98%; POV: 1.0 meq/kg or less) containing 4000 ppm of α-tocopherol, 20 parts of sample 1 obtained in Preparation Example 2, and 10 parts of casein sodium were emulsified in a high-speed mixer in a nitrogen atmosphere and spray dried to prepare a powdered drug prophylactic for habitual diseases (β-glucan content: 4.60%) according to the present invention. The β-glucans were found uniformly dispersed. The drug prophylactic for habitual diseases according to the present invention was evaluated for stability. The results are shown in Table 1. The POV of the powder was 0.8 meq/kg, proving the drug to be excellent in antioxidation stability.

Comparative Example 23: Production of drug prophylactic for habitual diseases

Three parts of high purity DHA (purity: 98%; POV: 1.0 meq/kg or less) containing 4000 ppm of α-tocopherol, 20 parts of water, and 10 parts of casein sodium were emulsified in a high-speed mixer in a nitrogen atmosphere and spray dried into powder, which was evaluated for stability for comparison. The results are shown in Table 2. The POV of the powder was 1.4 meq/kg, indicating inferior antioxidation stability.

TABLE 1

| Example No. | Stability | Texture | | |
|---|---|---|---|---|
| | | Smoothness | Hardness | Flavor |
| 1 | - | - | - | - |
| 2 | - | - | - | - |
| 3 | - | - | - | - |
| 4 | - | - | - | - |
| 5 | - | - | - | - |
| 6 | - | - | - | - |
| 7 | - | - | - | - |
| 8 | - | A | - | A |
| 9 | A | A | - | A |
| 10 | A | - | - | A |
| 11 | A | - | - | A |
| 12 | A | A | - | A |
| 13 | A | A | - | A |
| 14 | A | A | - | A |
| 15 | - | - | - | - |
| 16 | - | - | A | A |
| 17 | - | - | A | A |
| 18 | - | A | A | A |
| 19 | - | A | A | A |
| 20 | - | - | A | A |
| 21 | - | - | A | A |
| 22 | - | - | A | - |
| 23 | - | A | - | - |
| 24 | - | A | - | A |
| 25 | - | A | A | A |
| 26 | - | A | - | A |
| 27 | - | A | - | A |
| 28 | A | A | - | A |

(continued)

| Example No. | Stability | Texture | | |
|---|---|---|---|---|
| | | Smoothness | Hardness | Flavor |
| 29 | A | A | - | A |
| 30 | A | - | - | A |
| 31 | - | A | - | A |
| 32 | A | - | - | - |

TABLE 2

| Comparative Example No. | Stability | Texture | | |
|---|---|---|---|---|
| | | Smoothness | Hardness | Flavor |
| 1 | - | B | - | C |
| 2 | A | A | - | B |
| 3 | B | - | - | B |
| 4 | B | - | - | A |
| 5 | C | A | - | B |
| 6 | C | B | - | B |
| 7 | B | A | - | B |
| 8 | - | - | B | B |
| 9 | - | - | B | B |
| 10 | - | A | B | B |
| 11 | - | A | B | B |
| 12 | - | - | B | A |
| 13 | - | - | B | A |
| 14 | - | - | B | - |
| 15 | - | A | - | B |
| 16 | - | A | B | C |
| 17 | - | A | - | C |
| 18 | - | A | - | C |
| 19 | B | A | - | B |
| 20 | B | - | - | C |
| 21 | B | - | - | B |
| 22 | - | B | - | C |
| 23 | C | - | - | - |

Examples 33 to 37 and Comparative Examples 24 to 27:

These Examples and Comparative Examples were carried out with the β-glucan extract (sample 2) which was prepared in Preparation Example 3 (Preparation of β-glucans).

Example 33:

In 50.5% of warm water (60°C) was dispersed 0.5% of the β-glucan extract (sample 2), and 49% of palm oil at 60°C was dispersed and emulsified therein. The resulting emulsion was homogenized at 60°C under a pressure of 100 kg/cm$^2$ and heated up to 100°C by use of a scraped surface heat exchanger (rotational speed: 1200 rpm). The emulsion was cooled to 5°C and aged for 24 hours to prepare an oil-in-water emulsified composition. When the resulting oil-in-water emulsified composition was stirred in a mixer, heated to 60°C, and centrifuged at a rotational speed of 3000 rpm for 20 minutes, it underwent no oil separation, showing high emulsion stability against both physical and thermal stresses.

Example 34:

In 49.5% of warm water (60°C) having dissolved therein 1% of a whey protein concentrate (mainly comprising β-lactalbumin and α-lactalbumin; protein content: 80%) was dispersed 0.5% of the β-glucan extract (sample 2), and 49% of palm oil heated at 60°C was dispersed and emulsified therein. The resulting emulsion was homogenized at 60°C under a pressure of 100 kg/cm$^2$ and heated up to 100°C by use of a scraped surface heat exchanger (rotational speed: 1200 rpm). The emulsion was cooled to 5°C and aged for 24 hours to prepare an oil-in-water emulsified composition. When the resulting oil-in-water emulsified composition was stirred in a mixer, heated to 60°C, and centrifuged at a rotational speed of 3000 rpm for 20 minutes, it underwent no oil separation, showing high emulsion stability against both physical and thermal stresses.

Comparative Example 24:

An oil-in-water emulsified composition was prepared in the same manner as in Example 33, except that the β-glucan extract (sample 2) as used in Example 33 was not added and that the warm water was used in a proportion of 51%. When the resulting oil-in-water emulsified composition was stirred in a mixer under the same condition as in Example 33, the oily component separated. When it was heated to 60°C and centrifuged at 3000 rpm for 20 minutes, phase separation showing marked oil separation occurred. Emulsion stability against physical and thermal stresses was not observed.

Comparative Example 25:

An oil-in-water emulsified composition was prepared in the same manner as in Example 33, except for adding 0.5% of a sucrose fatty acid ester (HLB: 16) in place of the β-glucan extract (sample 2) used in Example 33. The composition was evaluated for emulsion stability in the same manner as in Example 33. When the resulting oil-in-water emulsified composition was stirred in a mixer, heated to 60°C, and centrifuged at 3000 rpm for 20 minutes, it underwent appreciable oil separation, showing no emulsion stability against physical and thermal stresses.

Comparative Example 26:

An oil-in-water emulsified composition was prepared in the same manner as in Example 33, except for adding 0.5% of a glycerol fatty acid ester (HLB: 4.3) in place of the β-glucan extract (sample 2) used in Example 33. The composition was evaluated for emulsion stability in the same manner as in Example 33. When the resulting oil-in-water emulsified composition was stirred in a mixer, heated to 60°C, and centrifuged at 3000 rpm for 20 minutes, it underwent appreciable oil separation, showing no emulsion stability against physical and thermal stresses.

Example 35:

In 56.6% of warm water (60°C) was dispersed 0.4% of the β-glucan extract (sample 2), and 43% of palm oil heated at 60°C was dispersed and emulsified therein. The resulting emulsion was homogenized at 60°C under a pressure of 100 kg/cm$^2$ and heated up to 100°C by use of a scraped surface heat exchanger (rotational speed: 1200 rpm). The emulsion was cooled to 5°C and aged for 24 hours to prepare an oil-in-water emulsified composition. The resulting oil-in-water emulsified composition was frozen at -20°C for 24 hours. When the frozen oil-in-water emulsified composition was thawed at 15°C, oil separation did not occur, proving the composition to have high emulsion stability against freezing.

Comparative Example 27:

An oil-in-water emulsified composition was prepared in the same manner as in Example 35, except that the β-glucan extract (sample 2) as used in Example 35 was not added and that the warm water was used in a proportion of 57%. The resulting oil-in-water emulsified composition was frozen under the same conditions as in Example 35. When the frozen oil-in-water emulsified composition was thawed at 15°C, it underwent oil separation, showing no emulsion stability against freezing.

Example 36:

Salted egg yolk (edible salt content: 8%) was adjusted to pH 8.4 with sodium hydroxide. To 100 parts of the salted egg yolk was added 0.015 part of phospholipase A of swine pancreatic juice origin, and the mixture was treated at 40°C for 6 hours. Then 0.001 part of bromelain was added and allowed to react at 45°C for 5 hours, followed by cooling to 10°C to obtain enzyme-processed egg yolk having a water content of 46%. Water (41%), 10% of thick malt syrup (water content: 30%), 7% of vinegar (acetic acid acidity: 10%; water content: 90%), 1.8% of edible salt, 0.1% of sodium glutamate, 0.1% of animal protein hydrolyzate, 0.5% of mustard powder, and 10% of the enzyme-processed egg yolk were mixed up to prepare an aqueous phase. Separately, an oily phase was prepared by mixing 27% of soybean salad oil, 1.5% of the β-glucan extract (sample 2), and 1% of modified starch obtained by phosphoric acid-crosslinking waxy corn followed by gelatinization. The oily phase was added to the aqueous phase while stirring to obtain an oil-in-water preliminary emulsion, which was emulsified in a colloid mill to obtain a sour oil-in-water emulsion containing the β-glucans of the present invention and having a water content of 55%. A 50 g portion of the sour oil-in-water emulsion was put into a 100 ml beaker and heated in a microwave oven (high-frequency output: 500 W) for 30 seconds, and the appearance, texture, and flavor were examined. It was found as a result that the emulsion underwent no oil separation, exhibited good shape retention, and had a creamy texture and a good mayonnaise flavor. Further, another 50 g portion of the sour oil-in-water emulsion was put into a 100 ml beaker, preserved in a freezer at -20°C for 30 days, and heated as frozen in a microwave oven (high-frequency output: 500 W) for 30 seconds, and the appearance, texture, and flavor were investigated. It was found as a result that the emulsion showed no oil separation, exhibited good shape retention, and had a creamy texture and a satisfactory mayonnaise flavor.

Example 37:

A mixed oil (81.3%) consisting of 80% of hydrogenated fish oil having a melting point of 34°C, 15% of soybean oil, and 5% of soft palm oil (melting point: 25°C) was mixed with 0.1% of lecithin and 0.1% of the β-glucan extract (sample 2), and the mixture was heated to about 60°C to prepare an oily phase. Separately, an aqueous phase was prepared from 16% of water, 1% of defatted milk powder, 1% of an enzyme-processed egg yolk prepared in the same manner as in Example 36, and 0.5% of edible salt. The aqueous phase was slowly added to the oily phase while stirring thoroughly to make a water-in-oil emulsion, which was sterilized and rapidly cooled for plasticization in a conventional manner to obtain margarine for dough and batter. The resulting margarine for dough and batter was evaluated for emulsion stability (whether water was released when aged at 5°C for 24 hours and then pressed with a spatula) and flavor. As a result, water separation was not observed, and the flavor was good.

Examples 38 to 42 and Comparative Example 28:

Quality modifiers were prepared by using the β-glucan extract (sample 2) obtained in Preparation Example 3 (Preparation of β-glucans). Ingredients were compounded according to the formulation shown in Table 3 below and mixed in a 10 liter rocking mixer, a dry powder blender, for 1 hour to obtain modifiers-1 to -5 and comparative product-1 having the ingredients uniformly dispersed.

The resulting modifiers-1 to -5 and comparative product-1 were subjected to a breadmaking test (Examples 38 to 42 and Comparative Example 28). The formulations and the steps used in the test are shown in Tables 4 and 5, respectively, and the test results are shown in Table 6.

Evaluation for the properties of dough and the quality of crumb and crust, texture, softness, and flavor of bread was made according to the following standards.

1) Dough properties

    A ... Not sticky
    B ... Slightly sticky

2) Crumb quality - observed with the naked eye

    A ... Fine cells
    B ... Having slightly rough cells

3) Crust quality - observed with the naked eye

> A ... Free from cracks, bumps, and roughness
> B ... Slight cracks, bumps or roughness observed

4) Texture
The texture was evaluated by 10 panel members and rated A or B according to the standard shown below. The rating given by the greater number out of 10 panel members was the rating of the sample.

> A ... Very good texture
> B ... Good texture

5) Softness
The softness was evaluated by 10 panel members and rated A or B according to the standard shown below. The rating given by the greater number out of 10 panel members was the rating of the sample.

> A ... Very soft
> B ... Soft

6) Flavor
The flavor was evaluated by 10 panel members and rated A or B according to the standard shown below. The rating given by the greater number out of 10 panel members was the rating of the sample.

> A ... Very good flavor
> B ... Good flavor

TABLE 3

| Formulation of Quality Modifier (general dough) | | | | | | |
|---|---|---|---|---|---|---|
| | Modifier | | | | | Comparative Product 1 |
| | 1 | 2 | 3 | 4 | 5 | |
| Extracted β-glucan | 85 | 70 | 60 | 80 | 90 | |
| Diacetyltartaric acid monoglyceride Na salt | | 10 | 15 | | 8 | 20 |
| Sucrose fatty acid ester | | | 1 | | | 12 |
| Fatty acid monoglyceride | | | | | | 8 |
| L-Ascorbic acid | | 0.2 | 0.3 | 0.7 | | 0.7 |
| L-Cystine | | | 0.1 | | | 0.2 |
| α-Amylase | | 0.5 | | 0.7 | 0.4 | 1 |
| Glucoamylase | | | | | | 0.5 |
| Hemicellulase | | 0.5 | | | 0.2 | 0.5 |
| Protease | | | | | | 0.1 |
| Wheat flour | 15 | 15 | 21.6 | 16.6 | | 54 |
| Sugar | | 3.8 | 2 | 2 | 1.4 | 3 |
| Total (%) | 100 | 100 | 100 | 100 | 100 | 100 |

TABLE4

| Formulation for Breadmaking by Sponge-Dough Process | | | | | | |
|---|---|---|---|---|---|---|
| | | | | | | (unit: part) |
| | Example | | | | | Comparative Example 28 |
| | 38 | 39 | 40 | 41 | 42 | |
| Bread flour (Eagle) | 70 | 70 | 70 | 70 | 70 | 70 |
| Yeast | 2 | 2 | 2 | 2 | 2 | 2 |
| Water | 46 | 46 | 46 | 46 | 46 | 46 |
| Bread flour | 30 | 30 | 30 | 30 | 30 | 30 |
| Sugar | 4 | 4 | 4 | 4 | 4 | 4 |
| Edible salt | 2 | 2 | 2 | 2 | 2 | 2 |
| Shortening | 4 | 4 | 4 | 4 | 4 | 4 |
| Defatted milk powder | 2 | 2 | 2 | 2 | 2 | 2 |
| Modifier-1 | 2 | | | | | |
| Modifier-2 | | 2 | | | | |
| Modifier-3 | | | 2 | | | |
| Modifier-4 | | | | 2 | | |
| Modifier-5 | | | | | 2 | |
| Comparative product- 1 | | | | | | 2 |
| Water | 25 | 24 | 24 | 25 | 25 | 20 |

TABLE 5

| Steps of 70% Sponge-Dough Process | | |
|---|---|---|
| Sponge | Mixing | Mixer: Shinagawa, Type 5 DM, L: 141 rpm; H: 2385 rpm 24°C; 15 mins. at low speed; 2 mins. at high speed |
| | Fermentation | 28°C, 30 mins. |
| Dough | Mixing | 28°C; 1.5 mins. at low speed; 2 mins. at high speed |
| Floor time | | 28°C, 30 mins. |
| Shaping | Dividing | Fold into three and divided into 450 g portions |
| | Rounding | The dough was passed through National Sheeter-moulder (roll clearance: 3/8 in.), folded into three, passed through the sheeter, again folded into three and again sheeted. |
| | First proofing | 28°C, 15 mins. |
| | Shaping | The dough was passed through the sheeter section of National Sheeter-moulder once at a roll clearance of 3/8 in. and then once at 7/32 in. in the same direction and finally at 1/8 in. in the opposite direction. The flattened dough was rolled up in the moulder section from its dry end making 15 turns. |
| | Shape | One-loaf |
| | Pan | One-loaf pan (top: 9.8 x 20.5 cm; bottom: 8.5 x 18.9 cm; 8.0 cm deep; 1445 cc) |
| | Final proofing Final proofing | 38°C, >90% RH, until the dough rose 2.0 cm above the lip of the pan |

(continued)

| Steps of 70% Sponge-Dough Process | | |
|---|---|---|
| Baking | Baking | 227 to 280°C, 23 mins. |
| | Cooling | Room temperature, 40 mins.; sealed into a polyethylene bag after cooling |

TABLE 6

| | Examples | | | | | Comparative Example 28 |
|---|---|---|---|---|---|---|
| | 38 | 39 | 40 | 41 | 42 | |
| Water absorption (%) | 71 | 70 | 70 | 71 | 71 | 66 |
| Dough properties | A | A | B | B | B | B |
| Volume (ml) | 2640 | 2658 | 2620 | 2651 | 2638 | 2530 |
| Crumb | A | A | A | A | A | A |
| Crust | B | A | B | B | B | B |
| Texture | A | A | A | A | B | A |
| Softness | A | A | B | B | A | B |
| Flavor | A | A | A | A | A | B |

As is apparent from the test results shown in Table 6, the modifiers-1 to -5 containing the extracted β-glucans (Examples 38 to 42) manifest excellent bread quality improving effects compared with the comparative product-1 containing no β-glucans (Comparative Example 28). It is also seen that the modifiers-1 to -5 contribute to flavor enhancement and enable reduction of amounts of additives conventionally used in breadmaking without while retaining the bread quality (Examples 39 to 42).

Examples 43 to 47 and Comparative Example 29:

Quality modifiers were prepared by using the β-glucan extract (sample 2) obtained in Preparation Example 3 (Preparation of β-glucans).
Ingredients were compounded according to the formulation shown in Table 7 below and mixed in a 10 liter rocking mixer, a dry powder blender, for 1 hour to obtain modifiers-6 to -10 and comparative product-2 having the ingredients uniformly dispersed.
Frozen dough for butter rolls was prepared by using the modifiers-6 to -10 and the comparative product-2 and evaluated for breadmaking properties (Examples 43 to 47 and Comparative Example 29). The formulation in the test is shown in Table 8 below. The dough was prepared as follows. Ingredients other than the modifier and margarine were put into a mixer and mixed at a low speed for 2 minutes and at a medium speed for 5 minutes. The modifier and margarine were added, and mixing was continued at a low speed for 2 minutes and then at a medium speed for 4 minutes. The mixing temperature was 27°C. The dough was allowed to ferment at 28°C and 75% humidity for 30 minutes. The proofed dough was divided into 45 g portions. After a bench time of 15 minutes, the proofed dough was shaped, deep frozen at -38°C for 15 minutes, and stored in a freezer at -20°C. After 2, 4, 6 or 8 week storage, the frozen dough was thawed at 20°C, finally proofed at 38°C and 80% humidity, and baked in an oven at 200°C for 10 minutes to obtain butter rolls. After cooling, the butter rolls were packed in a polyethylene bag. After left to stand at 25°C for 24 hours, the butter rolls were evaluated for specific volume, hardness, appearance, and taste. The results obtained are shown in Table 9 below.
The specific volume is a value obtained by dividing the volume of a sample by its weight. The higher the value, the higher the volume. The hardness was represented by the number of grams required for 40% compressing a 3 cm thick cut piece of a sample with a disk of 2.5 cm in diameter as measured with a rheometer (Yamaden Incorporation). The smaller the number, the softer. The appearance was observed with the naked eye and ranked A to C (A: even brown color with a sheen; B: slightly uneven brown color with a poor sheen; C: considerably uneven brown color with no sheen). The taste was ranked A to C (A: excellent in flavor and softness; B: normal; C: lacking in flavor or softness).

TABLE 7

| Formulation of Quality Modifier (frozen dough) | | | | | | |
|---|---|---|---|---|---|---|
| | Modifier | | | | | Comparative Product 2 |
| | 6 | 7 | 8 | 9 | 10 | |
| Extracted β-glucans | 85 | 73 | 60 | 80 | 87 | |
| Diacetyltartaric acid monoglyceride Na salt | | 15 | 18 | | 8 | 20 |
| Sucrose fatty acid ester | | | 1 | | | 15 |
| Fatty acid monoglyceride | | | | | | 15 |
| L-Ascorbic acid | | 0.7 | 0.5 | 1 | | 1 |
| L-Cystine | | | 0.2 | | | 1 |
| α-Amylase | | 0.5 | | 0.5 | 0.3 | 0.6 |
| Glucoamylase | | 0.2 | | 0.1 | 0.3 | |
| Hemicellulase | | 0.4 | | 0.4 | 0.2 | 0.4 |
| Protease | | | | | | 0.2 |
| Activated wheat gluten | 8 | 9.2 | 15 | 6 | 4.4 | 20 |
| Wheat flour | 7 | | 3.3 | 10.1 | | 24 |
| Sugar | | 1 | 2 | 2 | | 2.5 |
| Total (%) | 100 | 100 | 100 | 100 | 100 | 100 |

TABLE 8

| Formulation of Frozen Dough (unit: part) | | | | | | |
|---|---|---|---|---|---|---|
| | Example | | | | | Comparative Example 29 |
| | 43 | 44 | 45 | 46 | 47 | |
| Bread flour | 100 | 70 | 70 | 70 | 70 | 70 |
| Yeast | 4 | 2 | 2 | 2 | 2 | 2 |
| Yeast food | 0.1 | | | | | |
| Water | 44 | 46 | 46 | 46 | 46 | 46 |
| Soft white sugar | 12 | 4 | 4 | 4 | 4 | 4 |
| Whole egg | 10 | | | | | |
| Edible salt | 1.3 | 2 | 2 | 2 | 2 | 2 |
| Margarine | 10 | 4 | 4 | 4 | 4 | 4 |
| Defatted milk powder | 2.5 | 2 | 2 | 2 | 2 | 2 |
| Modifier-6 | 4 | | | | | |
| Modifier-7 | | 4 | | | | |
| Modifier-8 | | | 4 | | | |
| Modifier-9 | | | | 4 | | |
| Modifier-10 | | | | | 4 | |
| Comparative Product-2 | | | | | | 4 |

TABLE 9

| Evaluation for Breadmaking Properties of Frozen Dough | | | | | | |
|---|---|---|---|---|---|---|
| | | Example | | | | | Comparative Example 29 |
| | | 43 | 44 | 45 | 46 | 47 | |
| 2 wks. | Specific volume | 5.4 | 5.5 | 5.3 | 5.4 | 5.5 | 5.1 |
| | Hardness | 68 | 67 | 69 | 69 | 71 | 81 |
| | Appearance | A | A | A | A | A | A |
| | Taste | A | A | A | A | A | B |
| 4 wks. | Specific volume | 5.1 | 5.5 | 5.3 | 5.2 | 5.4 | 4.8 |
| | Hardness | 73 | 70 | 75 | 71 | 77 | 89 |
| | Appearance | A | A | A | A | A | A |
| | Taste | A | A | A | A | A | B |
| 6 wks. | Specific volume | 5.1 | 5.1 | 5.2 | 5.1 | 4.9 | 4.5 |
| | Hardness | 79 | 78 | 80 | 82 | 81 | 92 |
| | Appearance | A | A | A | A | B | B |
| | Taste | A | A | A | A | B | C |
| 8 wks. | Specific volume | 4.6 | 4.9 | 5 | 4.8 | 4.7 | 4.4 |
| | Hardness | 85 | 79 | 82 | 85 | 87 | 110 |
| | Appearance | A | A | A | A | B | B |
| | Taste | A | A | A | B | B | C |

As is apparent from the results of evaluation shown in Table 9 above, the frozen dough containing the modifiers-6 to -10 containing the β-glucan extract and having been stored in a freezer for 2 to 8 weeks exhibited excellent breadmaking properties. It was proved that the modifiers are contributory to flavor enhancement and enable reduction of amounts of additives conventionally employed in bakery products while retaining breadmaking properties of dough.

Examples 48 to 53 and Comparative Examples 30 to 35:

Quality modifiers were prepared by using the β-glucan extract (sample 2) obtained in Preparation Example 3 (Preparation of β-glucans).

Quality modifying compositions for bakery products comprising a β-glucan extract, a glucide having at least one of an 1-2-α-D-glucopyranose bond, an 1-3-α-D-glucopyranose bond, an 14-α-D-glucopyranose bond, and an 1-6-α-D-glucopyranose bond (α-glucan), and D-fructose (Examples 48 to 53) were evaluated for volume, flavor, texture, and crispness. The results obtained are shown in Table 10 below.

Bread, French bread, Danish pastries, pizza crusts and biscuits were made in usual manners. The volume was evaluated with the naked eye. The flavor, texture, and crispness were evaluated by 10 panel members and rated on an A-to-C scale according to the following standards. The rating given to a sample by the greatest number out of the 10 panel members was the rating of the sample. For comparison, Comparative Examples 30 to 35 were carried out. In Table 10 below, the amounts of the extracted β-glucans, D-fructose, and α-glucans are represented as a percentage to wheat flour taken as 100%.

1) Volume

A ... Excellent volume

B ... Good volume

C ... Poor volume

2) Flavor

    A ... Very good flavor
    B ... Good flavor
    C ... Poor flavor

3) Texture

    A ... Very good texture
    B ... Good texture
    C ... Poor texture

4) Crispness

    A ... Very crispy
    B ... Crispy
    C ... Not crispy

TABLE 10

| | Example | | | | | | Comparative Example | | | | | |
|---|---|---|---|---|---|---|---|---|---|---|---|---|
| | 48 | 49 | 50 | 51 | 52 | 53 | 30 | 31 | 32 | 33 | 34 | 35 |
| Bakery product | bread | French bread | Danish pastry | pizza crust | bread | biscuits | bread | French bread | Danish pastry | pizza crust | bread | biscuits |
| Process | straight | - | - | - | sponge-dough | - | straight | - | - | - | sponge-dough | - |
| Extracted β-glucans (%) | 13.13 | 0.148 | 2.96 | 27.54 | 18.75 | 4.86 | - | - | - | - | - | - |
| D-Fructose (%) | 1.3 | 0.01 | 0.6 | 3.5 | 2.3 | 25 | 1.3 | 0.01 | 0.6 | 3.5 | 2.3 | 25 |
| α-Glucan (starch) content in flour (%) | 75 | 72 | 74 | 76 | 73 | 76 | 75 | 72 | 74 | 76 | 73 | 76 |
| α-Glucans added (%) | 0 | 2 | 0 | 5 | 2 | 5 | 0 | 2 | 0 | 5 | 2 | 5 |
| Total α-glucans (%) | 75 | 74 | 74 | 81 | 75 | 81 | 75 | 74 | 74 | 81 | 75 | 81 |
| Extracted β-glucans/D-fructose | 10.1 | 14.8 | 4.9 | 7.9 | 8.2 | 0.19 | - | - | - | - | - | - |
| Extracted β-glucans/total α-glucans | 0.175 | 0.002 | 0.04 | 0.34 | 0.25 | 0.06 | - | - | - | - | - | - |
| Yeast | used | used | used | used | used | not used | used | used | used | not used | used | not used |
| Volume | B | B | A | B | A | A | C | C | C | C | C | C |
| Flavor | B | A | A | B | A | B | C | C | C | C | C | C |

(continued)

| | Example | | | | | | Comparative Example | | | | | |
|---|---|---|---|---|---|---|---|---|---|---|---|---|
| | 48 | 49 | 50 | 51 | 52 | 53 | 30 | 31 | 32 | 33 | 34 | 35 |
| Texture | B | B | B | A | A | A | C | C | C | C | C | C |
| Crispness | - | - | - | A | - | A | - | - | - | C | - | C |

Example 54: Production of instant powdered consomme

The β-glucan extract (sample 2) obtained in Preparation Example 3 (Production of β-glucans) was mixed into a composition consisting of powdered soy sauce, edible salt, sugar, sodium glutamate, ginger powder, onion powder, fat powder, ground pepper, and beef-flavored powdered soup in such an amount as to give a 2% concentration when mixed with hot water to obtain instant powdered consomme. On pouring hot water into the instant powdered consomme, the powder was dispersed in hot water without forming lumps to give consomme containing β-glucans.

Example 55: Making of corn potage

A hundred milliliters of two-fold concentrate potage (available from Asahi Denka Kogyo K.K.), 100 ml of milk, and 20 g of the β-glucan extract (sample 2) obtained in Preparation Example 3 (Production of β-glucans) were mixed by stirring at 80°C to prepare β-glucan-containing potage. The corn potage of the present invention was excellent with no strange feeling in taste and texture, such as rough texture.

Example 56: Production of fermented lactic acid beverage

Fifteen grams of fermented milk having a milk solid content of 21%, 13 g of fructose/glucose liquid sugar, 0.5 g of pectin, 0.08 g of citric acid, 0.15 g of flavor, 2 g of the β-glucan extract (sample 2) obtained in Preparation Example 3 (Production of β-glucans), and 64 g of water were mixed by stirring to prepare a β-glucan-containing fermented lactic acid beverage. The fermented lactic acid beverage was excellent in uniformity, taste and texture.

Example 57: Making of milk beverage

One gram of the β-glucan extract (sample 2) obtained in Preparation Example 3 (Production of β-glucans) was mixed into 150 ml of milk by stirring to obtain a β-glucan-containing milk beverage. The β-glucans were found uniformly dissolved and dispersed in the beverage, and the milk beverage was excellent in taste and texture.

Example 58: Making of cocoa

The β-glucan extract (sample 2) obtained in Preparation Example 3 (Production of β-glucans) was mixed into a commercially available instant cocoa mix in such an amount as to give a 5% concentration when mixed with hot water to prepare a β-glucan-containing instant cocoa mix. On pouring hot water, the instant cocoa mix was dispersed without forming lumps to provide β-glucan-containing cocoa.

Example 59: Making of cream soup with onion

β-Glucan-containing powdered soup mix was prepared by uniformly mixing well 28.7 g of edible oil, 19 g of wheat flour, 12.52 g of dried onion chips, 12 g of defatted milk powder, 8 g of edible salt, 4 g of onion powder, 1.25 g of sodium glutamate, 2 g of chicken extract, 0.1 g of white pepper, 10 g of maize flour, 0.4 g of carrageenan, and 2.03 g of the β-glucan extract (sample 2) obtained in Preparation Example 3 (Production of β-glucans). A comparative soup mix was prepared by replacing the β-glucans with 2.03 g of wheat flour. On pouring 100 ml of hot water into 20 g of each soup mix, the β-glucan-containing soup mix was dissolved and dispersed uniformly in several seconds, whereas the comparative soup mix formed lumps and was difficult to dissolve.

Example 60: Making of *tempura* - 1

A hundred grams of commercial cake flour (Nippon Flour Mills Co., Ltd.), one whole egg, 3 g of the β-glucan extract (sample 2) obtained in Preparation Example 3 (Production of β-glucans), and 120 g of water were lightly mixed to prepare batter. Prawns from which the head and vein had been removed were each dipped in the batter and deep-dried in oil to make *tempura.* For comparison, *tempura* was made by using batter containing no β-glucans. The *tempura* of the present invention was superior to the comparative one in brown color, taste and texture.

Example 61: Making of *tempura* - 2

A hundred grams of commercial cake flour (Nippon Flour Mills Co., Ltd.), one whole egg, 3 g of the β-glucan extract (sample 2) obtained in Preparation Example 3 (Production of β-glucans), and 120 g of water were lightly mixed to prepare batter. Prawns from which the head and vein had been removed were each dipped in the batter and deep frozen at -20°C. The resulting frozen prawn *tempura* ready to deep-fry was preserved at -18°C for 1 week and deep-dried in oil. For comparison, *tempura* was made by using batter containing no β-glucans. The *tempura* of the present invention was superior to the comparative one in brown color, taste and texture.

Example 62: Making of *okonomi-yaki* (as-you-like-it pancake)

A hundred fifty grams of commercial cake flour (Nippon Flour Mills Co., Ltd.), 3/4 cup of water, 5 g of the β-glucan extract (sample 2) obtained in Preparation Example 3 (Production of β-glucans), and a pinch of edible salt were mixed up thoroughly, and one whole egg was mixed therein to prepare thin dough. A portion of the dough was spread on a hot plate oiled with the fat and oil composition containing the β-glucans extracted from a gramineous plant which was obtained in Example 4. Cabbage, green onion, *sakura* shrimps, and green seaweed flakes were placed thereon, and the rest of the dough was poured thereon. The dough with filling was turned up and further cooked to make *okonomi-yaki*. Comparative *okonomi-yaki* was made without using β-glucans. The *okonomi-yaki* of the present invention was excellent in brown color, taste, and texture.

Example 63: Production of frozen creamy crab *korokke* (Japanese style croquettes) - 1

A pan was put on fire, and 70 parts of cake flour, 70 parts of salted butter, and 80 parts of onion were sauteed in the pan. The pan was taken off the fire, and 360 parts of milk was added. The pan was put back on the fire, and the mixture was stirred well to prepare white sauce. A hundred parts of canned crab meat was mixed into 500 parts of the white sauce, and the mixture was shaped into patties each weighing 50 g. Each patty was covered with cake flour, egg, and then bread crumbs containing 5% of the β-glucan extract (sample 2) obtained in Preparation Example 3 (Production of β-glucans) and deep frozen at -30°C. After 1 month storage at -20°C, the frozen *korokke* was thawed, deep dried in salad oil heated to 180°C for 2 minutes and 30 seconds, drained on paper towel to make creamy crab *korokke*. Comparative creamy crab *korokke* was made without using the β-glucans. The creamy crab *korokke* of the present invention was superior to the comparative one in taste and texture.

Example 64: Production of frozen creamy crab *korokke* - 2

A pan was put on fire, and 70 parts of cake flour, 70 parts of salted butter, and 80 parts of onion were sauteed in the pan. The pan was taken off the fire, and 360 parts of milk was added. The pan was put back on the fire, and the mixture was stirred well to prepare white sauce. A hundred parts of canned crab meat was mixed into 500 parts of the white sauce, and the mixture was shaped into patties each weighing 50 g. Each patty was covered with cake flour, egg, and then bread crumbs containing 5% of the β-glucan extract (sample 2) obtained in Preparation Example 3 (Production of β-glucans), and deep dried in salad oil heated to 180°C for 2 minutes and 30 seconds. The resulting *korokke* was deep frozen and stored at -20°C. For comparison, frozen *korokke* was made without using the β-glucans. After 1 month storage, the frozen *korokke* was cooked in a microwave oven (500 W) for 1 minute. The creamy crab *korokke* according to the present invention was crispy, not watery, and excellent in appearance, flavor and texture as compared with the comparative one.

Industrial Applicability:

The present invention provides a β-glucan-containing fat and oil composition which supplies β-glucans having excellent bioregulatory functions without being accompanied by reductions in taste, texture, and the like.

**Claims**

1. A β-glucan-containing fat and oil composition **characterized by** containing β-glucans which have a molecular weight average of less than 500.000 and are extracted from a gramineous plant.

2. The β-glucan-containing fat and oil composition according to claim 1, which contains a β-glucan having at least two kinds of bonds selected from a 1-2-β-D-glucopyranose bond, a 1-3-β-D-glucopyranose bond, a 1-4-β-D-glucopyranose bond, and a 1-6-β-D-glucopyranose bond.

3. The β-glucan-containing fat and oil composition according to claim 1 or 2, which contains a β-glucan having a 1-3-β-D-glucopyranose bond and a 1-4-β-D-glucopyranose bond.

**4.** The β-glucan-containing fat and oil composition according to any one of claims 1 to 3, wherein said gramineous plant is barley or oats.

**5.** A food containing the β-glucan-containing fat and oil composition according to any one of claims 1 to 4.

**6.** A fat and oil emulsified composition containing the β-glucan-containing fat and oil composition according to any one of claims 1 to 4.

**7.** A bakery product containing the β-glucan-containing fat and oil composition according to any one of claims 1 to 4.

**8.** A confectionery product containing the β-glucan-containing fat and oil composition according to any one of claims 1 to 4.

**9.** A food having a prophylactic action for a habitual disease containing the β-glucan-containing fat and oil composition according to any one of claims 1 to 4.

**10.** A drug having a prophylactic action for a habitual disease containing the β-glucan-containing fat and oil composition according to any one of claims 1 to 4.

**11.** A processed rice, wheat, maize or soybean product containing the β-glucan-containing fat and oil composition according to any one of claims 1 to 4.

**12.** A liquid food containing the β-glucan-containing fat and oil composition according to any one of claims 1 to 4.

**13.** A processed food mainly comprising starch and containing the β-glucan-containing fat and oil composition according to any one of claims 1 to 4.


**Patentansprüche**

**1.** β-Glucan enthaltende Zusammensetzung aus Fett und Öl, **dadurch gekennzeichnet, dass** sie β-Glucane enthält, die einen Molekulargewichtsdurchschnitt von weniger als 500000 haben und aus einer Graspflanze extrahiert sind.

**2.** β-Glucan enthaltende Zusammensetzung aus Fett und Öl nach Anspruch 1, die ein β-Glucan mit wenigstens zwei Arten von Bindungen, ausgewählt aus einer 1-2-β-D-Glucopyranose-bindung, einer 1-3-β-D-Glucopyranosebindung, einer 1-4-β-D-Glucopyranosebindung und einer 1-6-β-D-Glucopyranose-bindung, enthält.

**3.** β-Glucan enthaltende Zusammensetzung aus Fett und Öl nach Anspruch 1 oder 2, die ein β-Glucan mit einer 1-3-β-D-Glucopyranosebindung und einer 1-4-β-D-Glucopyranosebindung enthält.

**4.** β-Glucan enthaltende Zusammensetzung aus Fett und Öl gemäß einem der Ansprüche 1 bis 3, wobei die Graspflanze Gerste oder Hafer ist.

**5.** Nahrung, enthaltend die β-Glucan enthaltende Zusammensetzung aus Fett und Öl gemäß einem der Ansprüche 1 bis 4.

**6.** Emulgierte Zusammensetzung aus Fett und Öl, enthaltend die β-Glucan enthaltende Zusammensetzung aus Fett und Öl gemäß einem der Ansprüche 1 bis 4.

**7.** Bäckereiprodukt, enthaltend die β-Glucan enthaltende Zusammensetzung aus Fett und Öl gemäß einem der Ansprüche 1 bis 4.

**8.** Süßwarenprodukt, enthaltend die β-Glucan enthaltende Zusammensetzung aus Fett und Öl gemäß einem der Ansprüche 1 bis 4.

**9.** Nahrung mit einer prophylaktischen Wirkung für eine Gewohnheitskrankheit (habitual disease), enthaltend die β-Glucan enthaltende Zusammensetzung aus Fett und Öl gemäß einem der Ansprüche 1 bis 4.

**10.** Arzneimittel mit einer prophylaktischen Wirkung für eine Gewohnheitskrankheit, enthaltend die β-Glucan enthaltende Zusammensetzung aus Fett und Öl gemäß einem der Ansprüche 1 bis 4.

**11.** Behandeltes Reis-, Weizen-, Mais- oder Sojabohnenprodukt, enthaltend die β-Glucan enthaltende Zusammensetzung aus Fett und Öl gemäß einem der Ansprüche 1 bis 4.

**12.** Flüssige Nahrung, enthaltend die β-Glucan enthaltende Zusammensetzung aus Fett und Öl gemäß einem der Ansprüche 1 bis 4.

**13.** Behandelte Nahrung, die hauptsächlich Stärke beinhaltet und die β-Glucan enthaltende Zusammensetzung aus Fett und Öl gemäß einem der Ansprüche 1 bis 4 enthält.

**Revendications**

**1.** Composition d'huile et de graisses contenant des β-glucanes **caractérisée en ce qu'**elle contient des β-glucanes qui ont un poids moléculaire moyen inférieur à 500 000 et qui sont extraits d'une plante graminée.

**2.** Composition d'huile et de graisses contenant des β-glucanes selon la revendication 1, qui contient un β-glucane ayant au moins deux types de liaisons choisies à partir d'une liaison 1-2-β-D-glucopyranose, une liaison 1-3-β-D-glucopyranose, une liaison 1-4-β-D-glucopyranose et une liaison 1-6-β-D-glucopyranose.

**3.** Composition d'huile et de graisses contenant des β-glucanes selon la revendication 1 ou 2, qui contient un β-glucane ayant une liaison 1-3-β-D-glucopyranose et une liaison 1-4-β-D-glucopyranose.

**4.** Composition d'huile et de graisses contenant des β-glucanes selon l'une quelconque des revendications 1 à 3, dans laquelle ladite plante graminée est l'orge ou l'avoine.

**5.** Aliment contenant la composition d'huile et de graisses contenant des β-glucanes selon l'une quelconque des revendications 1 à 4.

**6.** Composition émulsifiée d'huile et de graisses contenant la composition d'huile et de graisses contenant des β-glucanes selon l'une quelconque des revendications 1 à 4.

**7.** Produit de boulangerie contenant la composition d'huile et de graisses contenant des β-glucanes selon l'une quelconque des revendications 1 à 4.

**8.** Produit de confiserie contenant la composition d'huile et de graisses contenant des β-glucanes selon l'une quelconque des revendications 1 à 4.

**9.** Aliment ayant une action prophylactique pour une maladie courante contenant la composition d'huile et de graisses contenant des β-glucanes selon l'une quelconque des revendications 1 à 4.

**10.** Médicament ayant une action prophylactique pour une maladie courante contenant la composition d'huile et de graisses contenant des β-glucanes selon l'une quelconque des revendications 1 à 4.

**11.** Produit traité de riz, blé, maïs ou soja contenant la composition d'huile et de graisses contenant des β-glucanes selon l'une quelconque des revendications 1 à 4.

**12.** Aliment liquide contenant la composition d'huile et de graisses contenant des β-glucanes selon l'une quelconque des revendications 1 à 4.

**13.** Aliment traité comprenant essentiellement de l'amidon et contenant la composition d'huile et de graisses contenant des β-glucanes selon l'une quelconque des revendications 1 à 4.